# EUROPEAN PATENT APPLICATION

(11) **EP 1 674 063 A1**
(43) Date of publication of application: **28.06.2006**
(21) Application number: 04030578.1
(22) Date of filing: 23.12.2004
(51) Int. Cl.: A61F 13/15

(54) **Extensible side panel**

(71) Applicant: 3M Innovative Properties Company, St. Paul, Minnesota 55133-3427 (US)
(72) Inventor: Petersen, Johann, 41516 Grevenbroich (DE); Selen, Peter, 5993 PG Maasbree (NL)
(74) Representative: Wilhelm, Stefan

(57) **Abstract**

An extensible side panel (10) for use in disposable articles such as a diaper (1), said panel comprising a first longitudinal side edge (11), a second longitudinal side edge (12), a first top lateral edge (13) and a second bottom lateral edge (14), the side panel comprising a first layer (30) of an extensible material (22) having a top lateral edge (30a) and a second layer (31) of an extensible material (22') having a top lateral edge (31a), said second layer (31) being superimposed onto said first layer (30) to provide at least one extensibly reinforced region (15), the top lateral edge or edges (31a) of the second layer (31) being arranged essentially parallel to the top lateral edge (30a) of the first (lower) layer of the side panel (10).

## Description

### Field of the invention

The present invention relates to an extensible side panel for use in disposable articles such as diapers, adult incontinence articles, sanitary napkins or training pants and to a method for manufacturing such extensible side panels. The invention also relates to disposable absorbent articles comprising at least one extensible side panel according to the invention.

### Background of the invention

Disposable absorbent articles can often be functionally separated into a chassis comprising an absorbent core to receive and contain urine and other body exudates, and side panels providing means for securing the disposable absorbent article to the wearer's body during use and preferably also extensible means to improve the fit of such disposable absorbent article on the wearer.

EP 0,888,101 discloses a method for manufacturing extensible side panels for disposable articles, the method comprising the steps of:
(a) providing a web of material in a machine direction, said web having a longitudinal centreline (L) generally parallel to said machine direction, a pair of longitudinal edges, designated final cut regions, a first region, a second region and a central zone, said central zone disposed between said first region and said second region;
(b) making a series of intermediate cuts in said web, each of said intermediate cuts extending across at least a portion of one of said first or second regions;
(c) folding said web along an axis that is generally parallel to said machine direction; and
(d) cutting said folded web in said designated final cut regions to produce individual extensible side panels.

The method is advantageous in that little or no scrap is produced but it is relatively complicated because it requires the application of intermediate cuts in both the first and second regions, the folding of the whole web in machine direction around the centre line and the application of cuts in the designated final cut regions.

The extensible side panels obtained in EP 0,888,101 exhibit an essentially wedge-shaped reinforced region where two layers of the material are superimposed on each other. The base of the essentially wedge-shaped reinforced region extends along the distal longitudinal edge of the side panel whereas its tip is typically arranged on the proximal longitudinal edge of the side panel. Such side panels exhibit multi-directional stretch characteristics but it is often preferred that the side panels impart stretch characteristics in particular in the direction of the waist area.

WO 97/47,265 and EP 0,808,145 disclose zero-scrap methods for manufacturing multi-directional extensible side panels comprising the steps of
(a) providing a web of material in a machine direction, said web having predetermined portions;
(b) activating said predetermined portions;
(c) making a continuous cut to sever said web into a first panel and a second panel, each of said panels having alternating, nested inwardly extending elements defined by said cut and terminating at a distal edge, said inwardly extending elements being offset in said machine direction;
(d) separating said first panel from said second panel;
(e) aligning said inwardly extending elements of said first panel and said second panel such that said distal edges of said inwardly extending elements are at least partially overlapping;
(f) joining at least a portion of said distal edge of said inwardly extending elements of said first panel with said distal edge of said inwardly extending elements of said second panel; and
(g) severing said first and said second panels at predetermined locations to create a side panel to be joined to said absorbent article.

The method includes applying a continuous cut to the web thus creating two panels. The elements of the two panels need to be aligned with respect to each other to provide multidirectional extensible side panels, which is less advantageous from a manufacturing point of view. The resulting extensible side panels are similar to those obtained in EP 0,888,101.

US 6,051,094 discloses a zero-scrap method of making a closure system for disposable articles. The method includes cutting of tab elements from a continuous closure system web which are then attached to an absorbent article web from which discrete disposable absorbent articles are eventually cut. The closure system tab elements provide both the fastening tabs and a suitable mating attachment surface for the disposable absorbent articles whereby the fastening tabs and mating attachment surface of a single closure system tab elements end up on different absorbent articles when these are cut from the absorbent article web. While US '094 provides a zero-scrap method for making a closure system for disposable articles it does not address the problem of providing extensible side panels having a non-uniform extensibility to improve the fit of the disposable absorbent article on the wearer.

The production of disposable absorbent articles is typically performed at high manufacturing speeds in order to lower the manufacturing cost per diaper. This requires that the method of production used is simple, highly reliable and easy to control. The manufacturing costs per diaper can also be lowered decreasing the amount of scrap obtained in the production process.

In view of the above, it is an object of the present invention to provide extensible side panels which allow for a reliable and pleasant fit of the diaper on the wearer and provide a high wearer comfort.

It is another object of the present invention to provide a method of manufacturing extensible side panels which can be run under high speed manufacturing conditions while producing little or no scrap or waste.

It is still another object of the present invention to provide extensible side panels suitable for use in disposable absorbent articles and a method for manufacturing such side panels which do not exhibit the shortcomings of the prior art or exhibit them to a lower degree only.

It is still another object of the present invention to provide disposable absorbent articles comprising extensible side panels contributing to a high wearer comfort of such disposable absorbent article.

Further objects of the present invention can be taken from the following detailed

### description of the invention.

### Summary of the invention

The present invention relates to an extensible side panel for use in disposable articles such as a diaper, said side panel comprising a first longitudinal side edge, a second longitudinal side edge, a first top lateral edge and a second bottom lateral edge, the side panel comprising a first layer of an extensible material having a top lateral edge and a second layer of an extensible material having a top lateral edge or edges, said second layer being superimposed onto said first layer to provide at least one extensibly reinforced region, the top lateral edge or edges of the second layer being arranged essentially parallel to the top lateral edge of the first layer of the side panel.

The present invention furthermore relates to a disposable absorbent article such as a diaper, a sanitary napkin or a training pant comprising a liquid permeable top sheet, a liquid-impermeable back sheet opposite to said top sheet, a liquid-absorbent core between said top sheet and said back sheet, longitudinal side edges and at least one extensible side panel according to the invention which is attached along at least one of the longitudinal side edges of the absorbent article.

The present invention furthermore relates to a method for preparing an extensible side panel according to the present invention comprising
(a) providing a web in a machine direction comprising a layer of an extensible material and a plurality of designated fold lines, said web having longitudinal side edges and said layer having a direction of extensibility essentially in the cross direction,
(b) making a series of intermediate cut lines in said web along a portion of the designated fold lines while maintaining the integrity of the web to provide foldable flaps adjacent to the designated fold lines,
(c) folding over said flaps around said designated fold lines and attaching the flap to the underlying web to provide extensibly reinforced regions, and
(d) cutting said web along the uncut portion of said designated fold lines to provide individual extensible side panels.

The present invention furthermore relates to another method for preparing an extensible side panel according to the present invention comprising
(a) providing a web in a machine direction comprising a layer of an extensible material, a plurality of longitudinal designated fold lines, longitudinal side edges and a plurality of designated cut lines in the cross direction, said layer having a direction of extensibility essentially in the machine direction,
(b) making a series of intermediate cut lines in said web along portions of the designated fold lines while maintaining the integrity of the web to provide foldable flaps adjacent to the designated fold lines,
(c) folding over said flaps around said designated fold lines and attaching the flap to the underlying web to provide extensibly reinforced regions,
(d) cutting said web along the remaining portions of said designated fold lines to provide a plurality of individual webs, and
(e) cutting said individual webs along said designated cut lines in the cross direction to provide individual extensible side panels.

The present invention furthermore relates to a method for preparing an extensible side panel according to the present invention comprising
(a) providing a web in a machine direction comprising a layer of an extensible material and a plurality of designated first top lateral edges, said web having longitudinal side edges and said layer having a direction of extensibility in the cross-direction,
(b) making a series of intermediate cut lines in said web along said designated first top lateral edges to provide a series of individual precursors of the extensible side panel comprising foldable flaps extending between the intermediate cut lines and the designated first top lateral edges and
(c) folding over said flaps around the designated first top lateral edge and attaching the flap to the first underlying layer to provide extensibly reinforced regions.

### Brief description of the figures

*Fig. 1a* is a schematic representation of a specific embodiment of the side panel 10 of the present invention comprising one elastically reinforced region 15 shown in hatched lines.
*Fig. 1b* is a schematic cross-sectional view along the line A―A in Fig. 1a.
*Fig. 1c* is a schematic cross-sectional view along the line B-B in Fig. 1 a.
*Fig. 1d* is a schematic representation of another specific embodiment of the side panel 10 of the present invention comprising two elastically reinforced regions 15.
*Fig. 1e* is a schematic representation of another specific embodiment of the side panel 10 of the present invention comprising one elastically reinforced region 15.
*Fig. 1f* provides a schematic representation of another specific embodiment of the side panel 10 of the present invention wherein the area of the elastically reinforced region 15 is smaller than the area of the hypothetical region 19 formed by the second bottom lateral edge 14 and the hypothetical cut line 18.
*Fig. 1g* provides a schematic representation of another specific embodiment of the side panel 10 of the present invention wherein the first top lateral edge 13 and the first (left) longitudinal edge 11 include an angle which is smaller than about 90°.
*Figs. 1h, 1i, 1j, 1k and 1l* are schematic representations of other specific embodiments of the extensible side panel 10 of the present invention which are similar to the embodiments of Figs. 1a, 1d, 1e, 1f and 1g, respectively. In the embodiments of Figs. 1h, 1j, 1k and 1l the top lateral edge of the reinforced layer 15 extends over the full width of the side panel 10. In the embodiment of Fig. 1i, the top lateral edge of the left reinforced layer 15 extends to the first (left) longitudinal edge 11 of the extensible side panel 10.
*Fig.* 2a is a schematic representation of a side panel 10 of the present invention additionally comprising a fastening member 16.
*Fig.* 2b is a schematic cross-sectional view along the line C-C in Fig. 2a.
*Fig.* 2c is a schematic representation of a side panel 10 of the present invention additionally comprising a fastening member 16 and two support layers 17.
*Fig.* 2d is a schematic cross-sectional view along the line D―D in Fig. 2c.
*Fig.* 2e provides a schematic representation of another embodiment of the side panels 10 of the present invention comprising fastening tabs 25 along the second longitudinal edge 12 and a support layer 17 along the first longitudinal edge 11.
*Figs.* 2f *and* 2g are schematic representations of other specific embodiments of the extensible side panel 10 of the present invention which are similar to the embodiments of Figs. 2a and 2e, respectively. In the embodiment of Fig. 2f, the top lateral edge of the reinforced layer 15 extends over the full width of the side panel. In the embodiment of Fig. 2g, the top lateral edge of the reinforced layer 15 extends to the first (left) longitudinal edge 11 of the extensible side panel 10.
*Figs.* 3a *and 3b* are schematic representations of diapers 100 comprising a chassis 110 and two extensible side panels 10 according to the present invention.
*Fig.* 4 is the schematic representation of a training pant 100 comprising a chassis 110 and two extensible side panels 10 according to the present invention.
*Figs.* 5a - 5c provide a schematic representation of a method of manufacturing extensible side panels 10 according to Fig. 1 h.
*Figs. 5d - 5f* provide a schematic representation of a method of manufacturing the extensible side panels 10 according to Fig. 1a.
*Figs.* 5g - 5i provide a schematic representation of a method of manufacturing the extensible side panels according to Fig. 2e.
*Figs. 5j - 5m* provide a schematic representation of a method of manufacturing the extensible side panels 10 of Fig. 2g.
*Figs.* 6a *and 6b* provide a schematic representation of another method of manufacturing the extensible side panels according to Fig. 1 a.
*Fig.* 6c provides a schematic representation of another method of manufacturing the extensible side panels 10 of Fig. 1 h.
*Figs.* 7a *and* 7b provide schematic illustrations of an apparatus suitable for manufacturing the extensible side panels of Fig. 1 a using a web 200 of an extensible material 22 having a direction of extensibility in CD.
*Fig.* 8 provides a schematic illustration of an apparatus suitable for manufacturing the extensible side panels of Fig. 1 h using a web 200 of an extensible material 22 having a direction of extensibility in CD.
*Fig.* 9 provides a schematic illustration of an apparatus suitable for manufacturing the extensible side panels of Fig. 1 h using a web 200 of an extensible material having a direction of extensibility in MD.
*Figs. 10a and 10b* show stress-strain curves for a sample cut out from the reinforced region 15 of side panels 10 obtained in Examples 1 and 2, respectively. Figs. 10a and 10b also show for comparative purposes stress-strain curves for samples cut out from the first (lower) layer 30 outside the reinforced region 15 of the side panels obtained in Examples 1 and 2, respectively.

### Detailed description of the invention

The present invention relates to an extensible side panel 10 suitable for use in disposable absorbent articles 100. As used above and below, the term "absorbent article" refers to articles which are placed against or in proximity to the body of the wearer to absorb and contain the various exudates discharged from the body. The absorbent article 100 preferably is disposable, i. e., it is intended to be disposed of after single use and not intended to be laundered or otherwise restored or reused.

A preferred embodiment of the absorbent article 100 referred to in the present invention is a diaper. The term "diaper" as used above and below refers to a garment generally worn by infants or incontinent persons that is drawn up between the legs and fastened around the waist of the wearer. Other embodiments of the absorbent article 100 include feminine hygiene garments such as sanitary napkins, training pants, incontinence briefs and diaper holders.

The term "extensible material" as used above and below means that the material extends at least in one direction when a force is applied. The term "the extensible material has a direction of extensibility essentially in a certain direction" means that the extensible material can be extended in such direction when applying a force essentially in such direction.

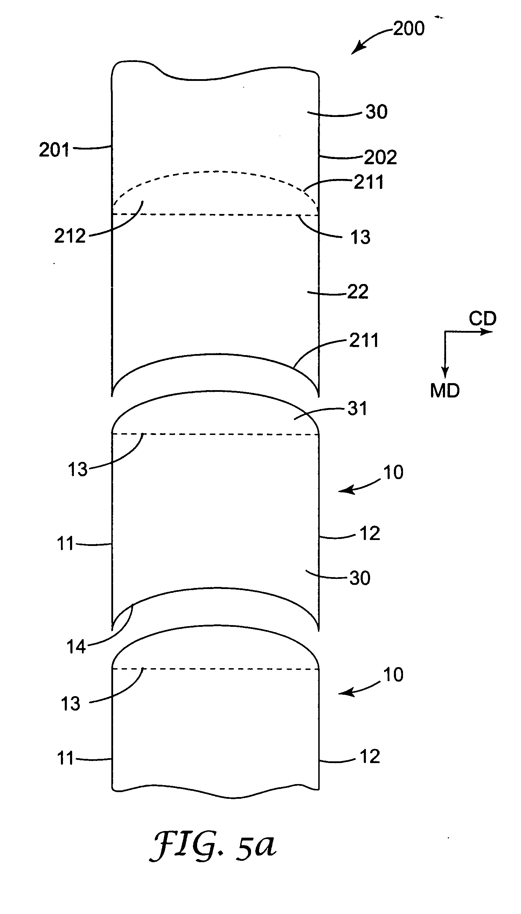

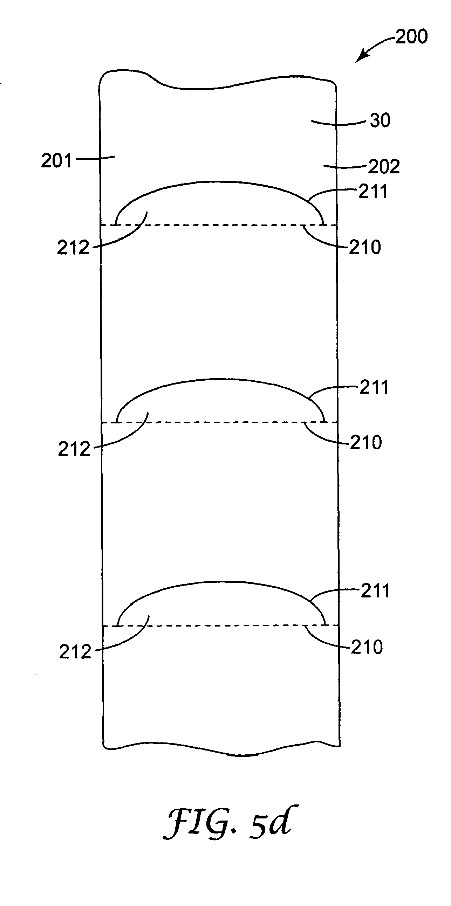

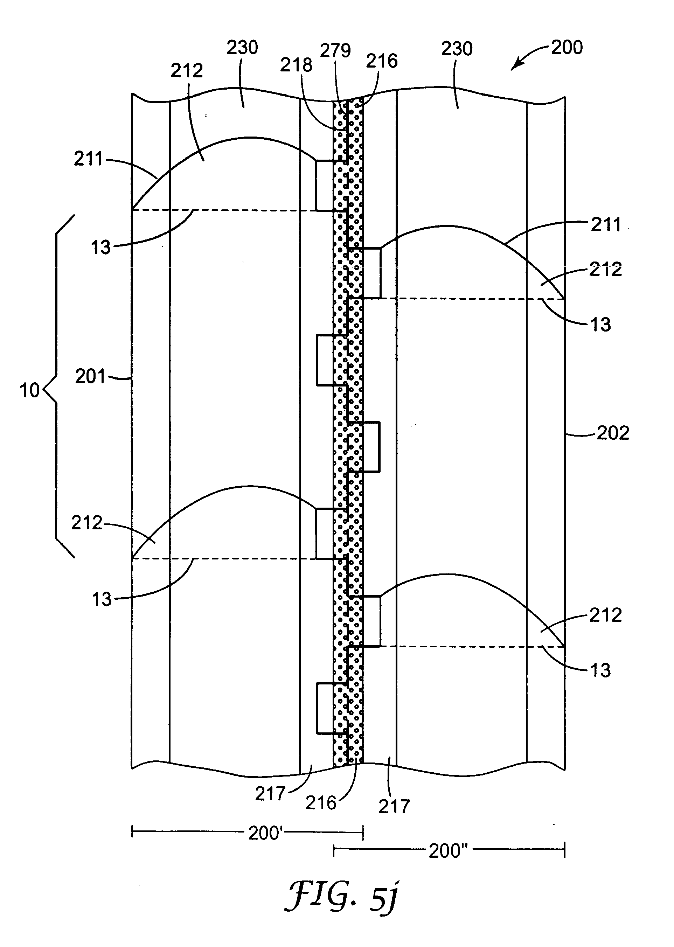

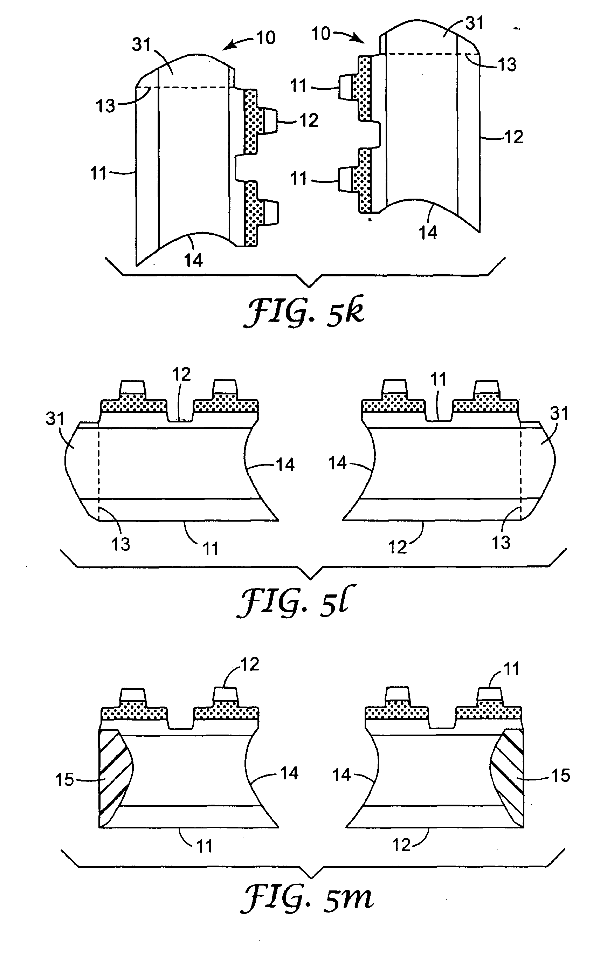

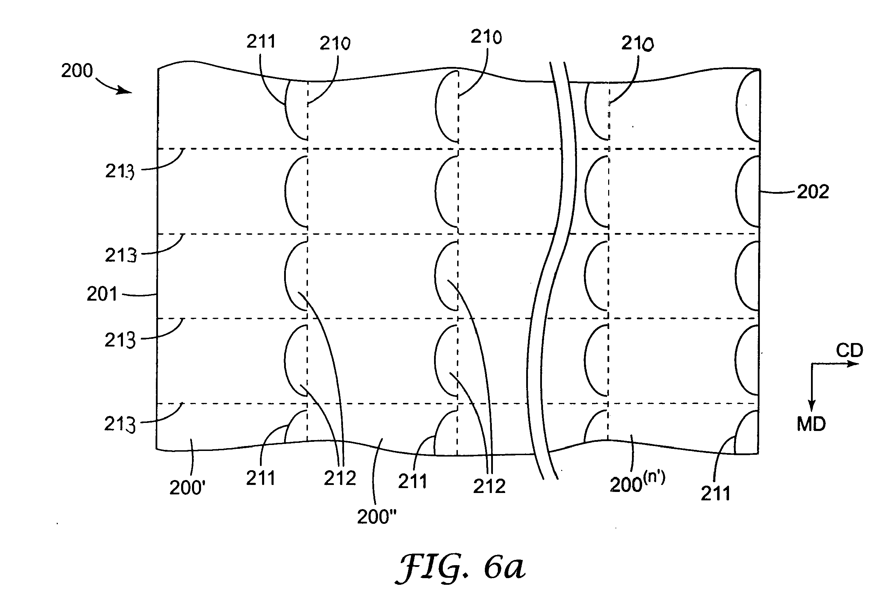

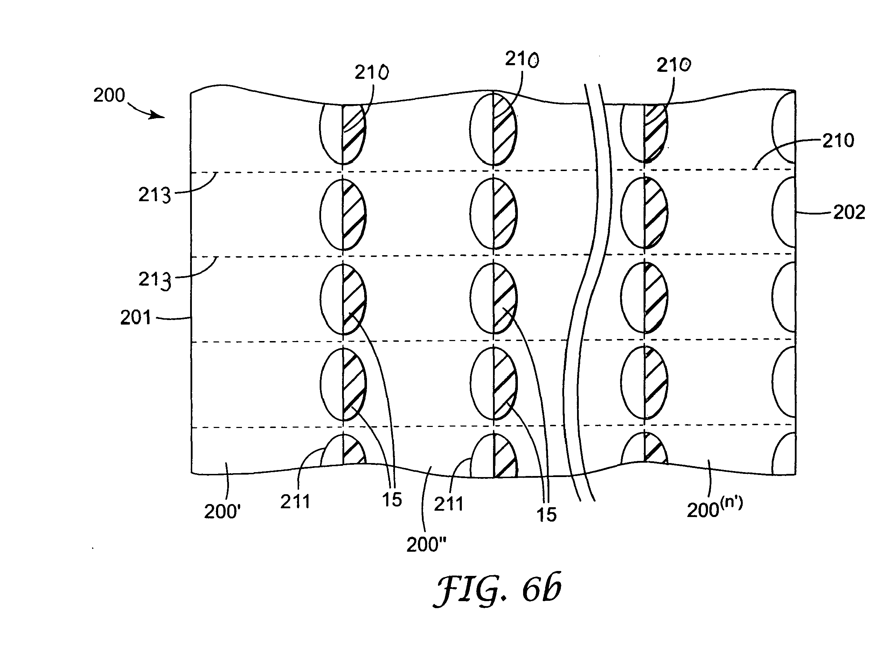

As used above and below, the term machine direction (MD) when used in connection with the web 200 denotes the direction of the running continuous web 200 comprising a web of an extensible material 22. The cross direction (CD) is essentially normal to the machine direction. MD and CD are indicated in Fig. 5a and 6a below.

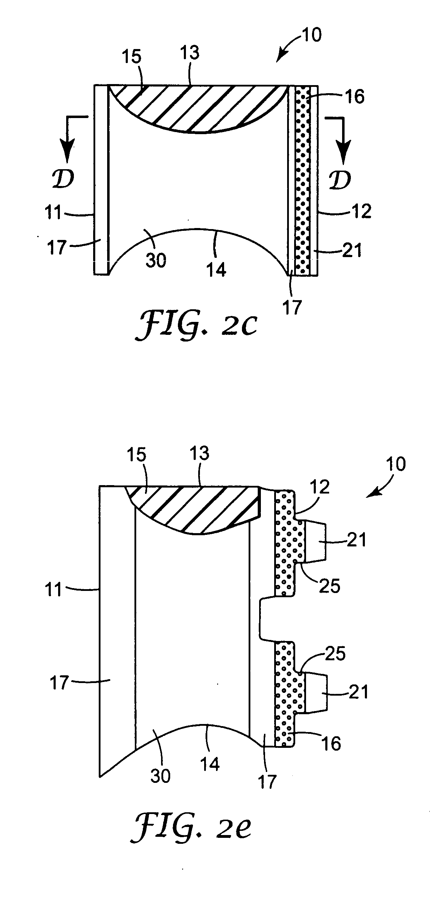

When used in connection with the side panels 10 of the present invention, the machine direction MD corresponds to the direction of the first and/or second longitudinal side edges 11, 12, respectively, of the extensible side panel 10. If the longitudinal edge or edges exhibit an irregular or contoured shape as exemplified for the second (right) longitudinal edge 12 in Fig. 2e, the MD is represented as the average direction along such edges. The cross-direction (CD) is essentially normal to MD. The CD also denotes a primary, i. e. reinforced direction of extensibility of the side panels 10.

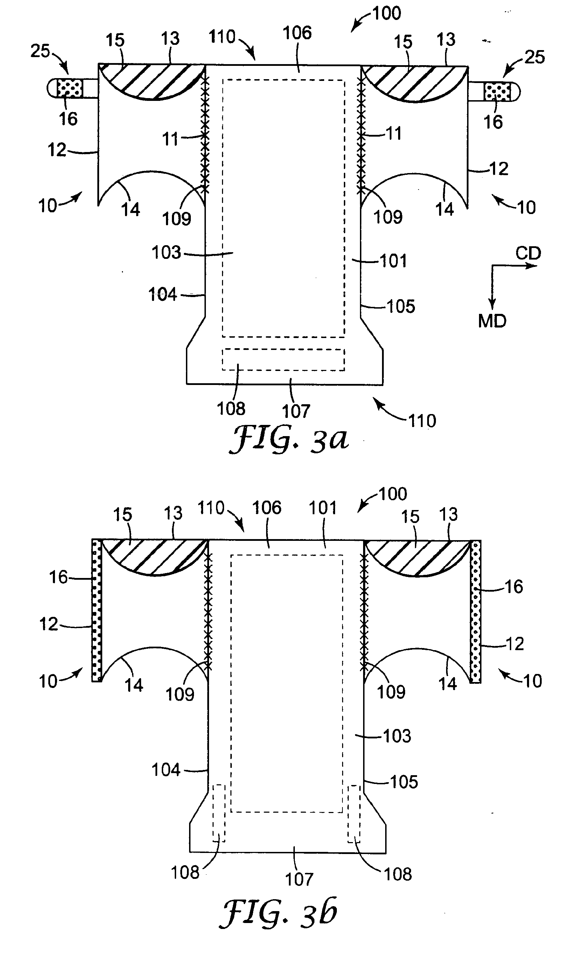

When used in connection with the disposable absorbent articles 100, the direction of the first and second longitudinal edges 104 and 105, respectively, is denoted as MD whereas the direction which is essentially normal to that direction is termed as CD. This is illustrated in Fig. 3a.

The present invention provides extensible side panels 10 having a first (left) longitudinal side edge 11 and a second (right) longitudinal side edge 12. The extensible side panel 10 comprises a first (lower) layer 30 of an extensible material 22 having a top lateral edge 30a. In the extensibly reinforced regions 15 of the side panel 10 a second (upper) layer 31 of an extensible material 22' having a top lateral edge 31a is superimposed onto at least part of said first layer. In the Figures shown below the reinforced regions 15 are shown in each case in hatched lines. The top lateral edge 13 of the side panel may be formed by the top lateral edge 30a and/or the top lateral edge(s) 31 a depending on whether the first (lower) layer 30 overshoots the second (upper) layer(s) 31 at the top edge of the side panel or vice versa. The side panel 10 is bordered at the lower edge by the bottom lateral edge 14.

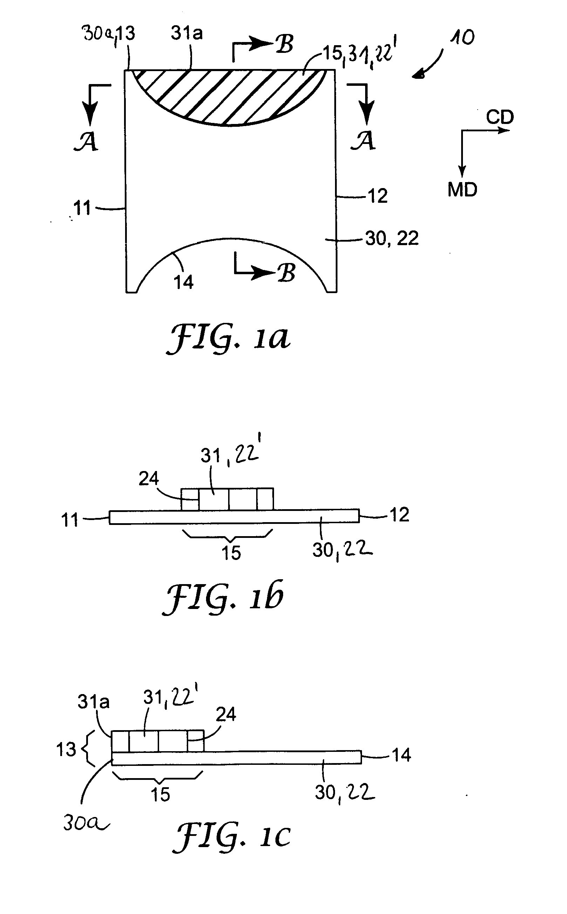

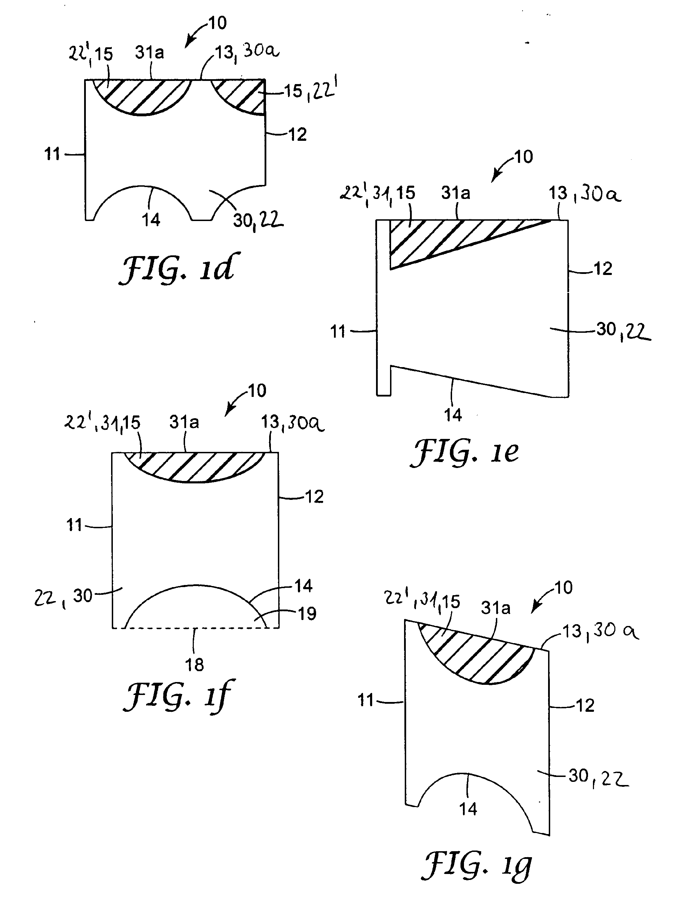

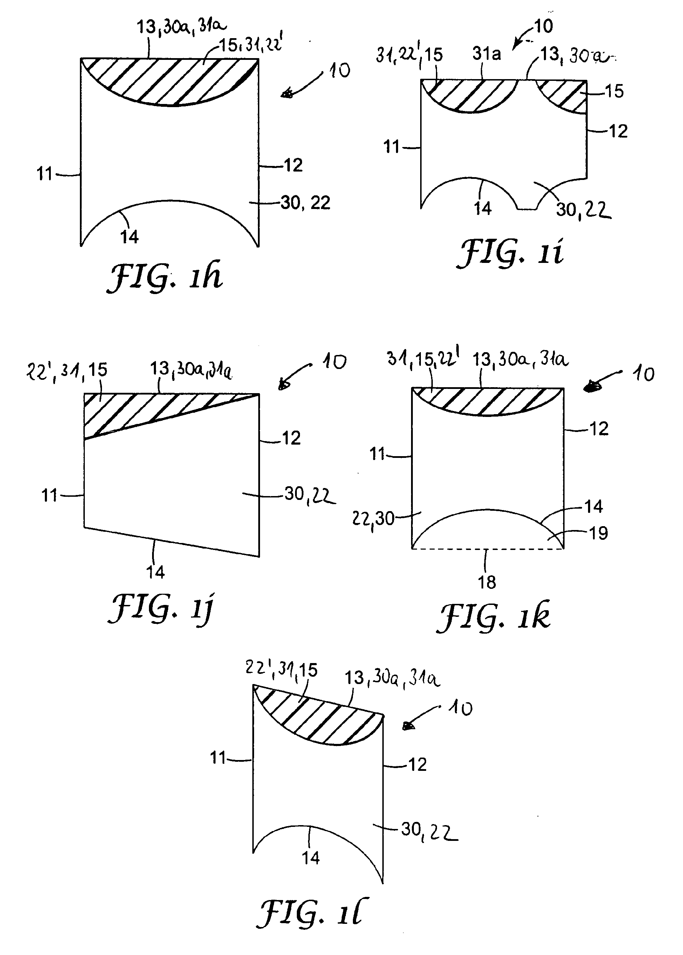

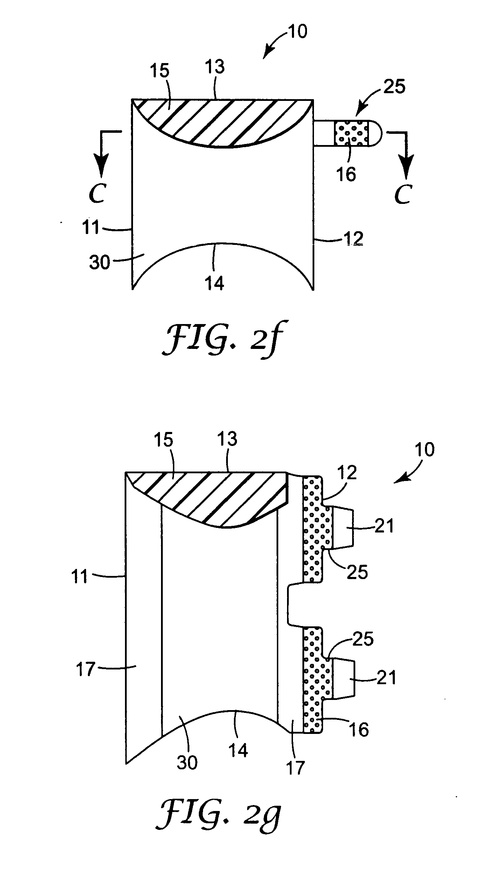

The extensible side panel 10 comprises one or more extensibly reinforced and, preferably, one or more elastically reinforced regions 15 where a second (upper) layer 31 comprising an extensible material 22' is superimposed onto at least part of said first (lower) layer 30 comprising an extensible material 22. This is shown, for example, in Figs. 1a-1c, where the second (upper) layer 31 is intermittently bonded to the first (lower) layer 30 by welded attachment lines 24 (see Figs. 1 b and 1c). In the embodiments of Figs. 2e and 2g, the second (upper) layer 31 is also attached to the support layers 17. The area of the extensibly reinforced region 15 is defined by the area of overlap between the first (lower) layer 30 and the second (upper) layer 31. The area of the reinforced region 15 can vary over a broad range but it is preferably less than 50 %, more preferably less than 35 % and especially preferably between 5-25 % of the area of the side panels 10 as defined by the first and second longitudinal side edges 11, 12, the first top lateral edge 13 and the second bottom lateral edge 14.

In the present invention the top lateral edge or edges 31a of the second (upper) layer or layers 31 comprising an extensible material 22', are arranged essentially parallel to the top lateral edge or edges 30a of the first (lower) layer 30. The term "essentially parallel" as used above and below is understood to denote that the top lateral edge or edges 31 a of the second overlying (upper) layer or layers 31 are arranged essentially in the direction of the top lateral edge or edges 30a of the first (lower) layer 30 so that the reinforced region 15 provides an increased retraction force (as compared to the retraction force of a hypothetical side panel having no second overlying layer 31) in the direction of the first top lateral edge 13 of the side panel 10, in the direction of the top lateral edge 30a of the first layer 30 and/or in the cross-direction. The term "essentially in the direction of the top lateral edge(s) 30a of the lower layer 30" as used above means that the directions of the top lateral edge or edges 30a of the first (lower) layer 30 and the directions of the top lateral edge(s) 31 a of the second (upper) layer 31 deviate from each other by less than 10°, more preferably by not more than 7.5° and especially preferably by less than 5°.

In a preferred embodiment the top lateral edge(s) 31a of the second (upper) layer(s) 31 comprising an extensible material 22' are arranged essentially adjacent and essentially parallel to the top lateral edge or edges 30a of the first (lower) layer 30 of the extensible side panel 10. The term "essentially adjacent" as used above and below is understood to denote that the top lateral edge(s) of the overlying second layer(s) 31 comprising an extensible material 22', are arranged in close proximity to the top lateral edge or edges 30a of the first (lower) layer 30. The average distance between the top lateral edge(s) of the overlying second layer(s) 31 and the top lateral edge(s) 30a of the first (lower) layer 30 preferably is less than 5 mm, more preferably not more than 3 mm and especially preferably less than about 1 mm.

In a preferred method of manufacturing side panels 10 according to the present invention illustrated, for example, in Figs. 5d - 5f below, the overlying layer 31 comprising the extensible material 22' is folded around the designated fold lines 210 so that the top lateral edge(s) 31 a of the overlying layer 31 essentially coincide with the top lateral edge(s) 30a of the first (lower) layer 30 to form the top lateral edge 13 of the side panel 10. This is illustrated, for example, for the side panel 10 of Fig. 1 a in the cross-sectional view along the line B-B shown in Fig. 1 c.

It is, however, also possible that the overlying layer 31 is provided as a separate piece of material 22' and is attached to the first layer 30 by any bonding method such as adhesion bonding, thermal bonding, ultrasonic bonding or mechanical bonding. In this case the top lateral edge(s) 31a of the overlying layer 31 and the top lateral edge(s) 30a of the first (lower) layer 30 of the side panel 10 may not coincide.

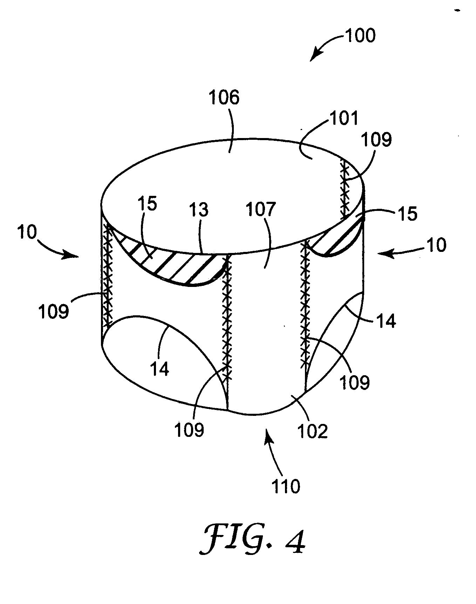

In a preferred use of the side panels of the present invention which is illustrated in Figs. 3a, 3b and 4 the side panel 10 is attached to the chassis 110 of a disposable absorbent article 100 so that the reinforced region 15 of the side panel 10 is arranged beside the back waistband region 106 and/or the front waistband region 107. It was found by the present inventors that the increase of the retraction force of the side panel in the reinforced regions 15 results in an improved fit of the diaper 100 on the wearer and in an increased wearer's comfort.

The underlying layer 30 and the overlying layer 31 within the reinforced region 15 both comprise extensible materials 22, 22' which may be the same or different from each other and/or exhibit the same or a different extensibility. In a preferred method of production of the extensible side panels 10 of the present invention which is illustrated, for example, in Figs. 5d - 5f below, a web 200 comprising a web of an extensible material 22 is provided in MD. The reinforced regions 15 are provided by making a series of discontinuous intermediate cut lines 211 into the web 200 thereby creating foldable flaps 212 which are folded around the designated fold lines 210. If the web 200 exhibits a uniform construction and composition in MD and requires an activation step to render it extensible and/or elastic, different extensibilities can be imparted to different regions of the web 200 in CD and/or MD by varying the extent of activation in CD and/or MD, for example, by using a ring-rolling activation technique. In the present invention it is preferred that the overlying layer 31 and the underlying layer 30 within the reinforced region 15 have the same composition but they may exhibit the same or a different activation treatment and thus the same or different extensibility properties. In an especially preferred embodiment, the first layer 30 and the second layer 31 comprise the same extensible material 22 having the same activation treatment.

In a preferred method of manufacturing the extensible side panels 10 of the present invention illustrated, for example, in Figs. 5a - 5c or 5d - 5f below, the area of the reinforced region 15 is essentially equal to or smaller than the area of the hypothetical region 19 which is formed by the second bottom lateral edge 14, a hypothetical cut line 18 which is parallel to the first top lateral edge 13 and extends through the lowest point of the second bottom lateral edge 14 and, optionally, a hypothetical extension of the first and/or second longitudinal edge 11, 12 in MD. This is exemplified in Fig. 1f below where the area of the hypothetical region 19 is larger than the area of the reinforced region 15. Preferably the area of the reinforced region 15 is essentially equal to the area of the hypothetical region 19 so that the method of preparing the extensibly reinforced side panel 10 is essentially wasteless. It is, however, also possible that the area of the reinforced region 15 is smaller than the area of the hypothetical region 19 as is illustrated, for example, in Figs. 1f and 1 k below.

The extensible side panel 10 may comprise one or more reinforced regions 15. An extensible side panel 10 comprising two reinforced regions 15 is shown, for example, in Fig. 1 d. Using two or more reinforced regions may be advantageous when using the side panels in disposable absorbent articles as illustrated in Figs. 3a, 3b and 4 in order to adjust the desirable retraction force exerted upon the back waistband region 106 and/or the front waistband region 107, respectively. It is preferred to use between 1 and 3 and, in particular, 1 or 2 reinforced regions 15. If the extensible side panel 10 exhibits 2 or more reinforced regions, the area of such regions is preferably selected so that the sum of the extensions of the lateral edges 31 a of the second (upper) layers 31 is at least 30 %, more preferably at least 50 % and especially preferably at least 75 % of the extension of the top lateral edge(s) 30a and/or of the first top lateral edge 13.

The increase of the retraction force in the direction of the first top lateral edge 13 of the side panel 10, in the direction of the top lateral edge 30a of the first layer 30 and/or in the cross-direction of the side panel 10 or the web 200, respectively, in comparison to a hypothetical construction where no upper layer 31 is present, mainly depends on the arrangement of the second (upper) layer 31 with respect to the first (lower) layer 30 and consequently on the arrangement of the top lateral edges 30a, 31 a with respect to each other, on the extensibility or elasticity properties, respectively, of the extensible materials 22' comprised in the upper layer 31 and on the area and shape of the reinforced region or regions 15. It is preferred in the present invention that such increase of the retraction force upon an elongation by 100 % preferably is at least 25 %, more preferably at least 50 % and especially preferably at least 75 %. The person skilled in the art can easily modify and adjust the modifying factors mentioned above in order to provide a desirable increase of the retraction force. The increase in the retraction force can be measured by recording stress-strain curves as is described in the sample section below for a sample cut from the reinforced region 15 by stretching such sample essentially in the direction of the first top lateral edge 13 of the side panel 10, in the direction of the top lateral edge 30a of the first layer 30 and/or in the cross-direction of the side panel 10 or the web 200, respectively. For comparison, a sample of the first (lower) layer 30 is also subjected to a stress-strain measurement by likewise stretching such sample essentially in the direction of the first top lateral edge 13 of the side panel 10, in the direction of the top lateral edge 30a of the first layer 30 and/or in the cross-direction of the side panel 10 or the web 200, respectively. The sample of the first (lower) layer 30 can be obtained in various ways. If the extensible material 22 of the web 200 is essentially uniform in MD and CD the sample can be cut from a different portion of the web 200 or the side panel 10. Alternatively, it may be possible to carefully remove the second (upper) layer 31 of a same second side panel and subsequently cut a sample from the exposed lower layer 30. In any case the comparative sample of the first (lower) layer 30 is obtained by applying a cut having the same geometry and orientation as the cut used to obtain the sample of the reinforced region 15. The increase of the retraction force for a specific elongation of, for example, 100% is obtained by comparing the force in the stress-strain diagram for the sample of the reinforced region with the force for the comparative sample of the first lower layer 30.

The extensible side panel 10 exhibits first (left) and second (right) longitudinal side edges 11, 12 which extend in MD of the side panel 10. These longitudinal edges 11, 12 may be essentially straight, parallel lines as is illustrated, for example, in Figs. 1 a and 1d - 1l. It is, however, also possible that one or both longitudinal edges 11, 12 are non-straight lines which may have an irregular or regular contour. In the embodiment of Fig. 2e the right longitudinal edge 12 of the side panel 10 exhibits a non-straight, meandering regular contour whereas the left longitudinal edge 11 is a straight line. It can be taken from Fig. 5i that the meandering longitudinal edge 12 of the side panel 10 of Fig. 2e is obtained by applying a regular periodic cut line 219 through and around the centre line 218 of the web 216 of a fastening member 16. In the specific embodiment of Fig. 5i the distance between two designated fold lines 210 (which defines the extension of the side panel 10 in MD) equals two times of the periodicity of the meandering cut line 219.

It is also possible that both of the first and second longitudinal side edges 11, 12 are non-straight lines, and they may be obtained in the methods of manufacturing described in more detail below, by applying non-straight irregular or regular cut lines to the web 200 in MD. Straight or non-straight, regular longitudinal side edges 11, 12 are preferred. The specific periodicity of the non-straight, regular longitudinal edge of Fig. 2e and 5i is an example only, and other periodicities may be chosen as well. In case both the first and second longitudinal side edge 11, 12 exhibit a non-straight, regular structure, the periodicities or wavelengths, respectively, of the two longitudinal edges 11 and 12 may be the same or different from each other.

The extensible side panel 10 furthermore exhibits a first top lateral edge 13 and a second bottom lateral edge 14. As was detailed above, the top lateral edges 30a, 31 a of first and second layers 30,31 preferably essentially coincide and form the first top lateral edge 13.

In a preferred method of production of the extensible side panels 10 illustrated, for example, in Figs. 5d - 5f below, the first top lateral edge 13 corresponds to the designated fold lines 210. The first top lateral edge 13 preferably forms an essentially straight line but other contours are also possible. The first top lateral edge 13 may extend essentially perpendicular to the MD as is shown, for example, in Figs. 1 a or 1h, but is also possible that the first top lateral edge forms an angle with the MD of the side panel 10 as is illustrated, for example, in Figs. 1g and 1l.

Such angle, if present, preferably is not less than 60° and, in particular, not less than 75° in order to provide a major component of the increased retraction force obtained through the reinforced regions 15, into the direction of the back or front waistband region 106, 107, respectively.

The extensible side panel 10 furthermore exhibits a second bottom lateral edge 14 which can have any contour including, for example, a straight line as is exemplified in Figs. 1 e and 1j, or a generally curved line as is exemplified in Figs. 1 a or 1 h. When using "big-ear"-type side panels 10 of the present invention in diapers (see Figs. 3a and 3b) or in training pants (see Fig. 4), the second bottom lateral edge 14 may encircle a leg of the wearer so that the contour of the second bottom lateral edge 14 is preferably selected in such constructions to provide for a high wearer's comfort. It may also be desirable that the reinforced region 15 is of an essentially rectangular shape to provide for an especially advantageous increase of the retraction force in the direction of the first top lateral edge 13 of the side panel 10, in the direction of the top lateral edge 30a of the first layer 30 and/or in the cross-direction of the side panel 10 or the web 200, respectively; in this case the bottom lateral edge 14 may be a straight line (if the rectangular reinforced region 15 extends over the full width of the side panel) or form an open rectangle, i.e. comprise 3 sides of a rectangle (if the rectangular reinforced region does not extend over the full width of the side panel).

The area between the first top lateral edge 13, the second bottom lateral edge 14 and the first and second longitudinal edges 11, 12 is denoted as the area of the side panel 10. The area of the side panel 10 may vary broadly depending on the application. In "big-ear"-type constructions the side panel may extend from the back waistband region 108 to the crotch region and may typically have an area of between 30 and 500 cm².

In other constructions where the side panel 10 provides, for example, an extensible or elastic fastening portion, the area of the side panel may be smaller and typically ranges between 15 and 100 cm². Such fastening portions which are not shown in the Figures below, preferably comprise in addition to the extensibly reinforced region 15 a fastening member 16. Other constructions and sizes of the side panels 10 are possible as well.

The side panels 10 of the present invention are preferably used in disposable absorbent articles 100 such as, for example, diapers or training pants.

The diaper 100 schematically shown in Fig. 3a comprises a chassis 110 and two side panels 10 which are attached to the first and second longitudinal edges 104, 105 of the diaper extending from the back waistband portion 106 of the diaper to the crotch area of the diaper. The chassis comprises an absorbent core 103 between a liquid-permeable top sheet 101 contacting the wearer's skin, and a liquid-impermeable back sheet 102 facing outwardly. The side panels 10 are secured to the chassis 110 in the attachment regions 109 by any bonding method including, for example, adhesive bonding, thermal bonding, ultrasonic bonding and/or mechanical attachment. In the diaper schematically shown in Fig. 3a, fastening tabs 25 bearing a fastening member 16 are attached to the distal longitudinal edges of the side panels 10.

The side panel 10 may comprise any fastening members 16 such as mechanical or adhesive fastening members. In case of adhesive fastening members a release-treated film is usually applied as a landing zone 108 to the front waistband region 107 of the diaper 100 to allow for releasably securing the diaper to the wearer. In case of mechanical fastening members, a landing zone 108 may be provided comprising a mechanical fastening member which is complementary to the mechanical fastening member arranged on the fastening tab 25. If a male (hook) mechanical fastening member is used on the fastening tab 25 and the back sheet 102 of the diaper 100 comprises an exposed non-woven layer, the male fastening member may directly interact with such non-woven layer and the landing zone 108 may be omitted. In the diaper schematically shown in Fig. 3b the fastening member 16 is attached to the layer 30 of an extensible material 22 so that no separate fastening tab 25 is required.

Fig. 4 schematically shows a training pant 100 where each of the two side panels 10 is attached through both of its respective longitudinal edges 11, 12 to the chassis 110 of the diaper to provide a pull-on disposable absorbent article.

The side panel or side panels 10, respectively, are preferably attached to disposable absorbent diapers such as, in particular, pull-up type training pants so that the extensibly reinforced region 15 is arranged in the area of the back waistband region 106 and/or the front waistband region 107. More preferably, the side panels 10 are arranged with respect to the diaper 100 so that the primary, i. e. reinforced direction of extensibility of such side panels 10 in the direction of the first top lateral edge 13, in the direction of the top lateral edge 30a of the first layer 30 and/or in the cross-direction of the side panel deviates from the CD of the back waistband region 106 of the diaper 100 and/or the CD of the front waistband region of the diaper 100 by less than 20°, more preferably by less than 15°, especially preferably by less than 10° and most preferably by less than 7.5°. In an especially preferred embodiment, the primary direction of extensibility of the side panels 10 deviates by not more than 5° from the CD of the back waistband region 106 of the diaper 100 and/or the CD of the front waistband region 107 of the diaper 100. It was found by the present inventors that diapers 100 comprising a higher degree of extensibility and/or elasticity and, consequently, higher retraction forces in the back waistband region 106 and/or front waistband region 107 and - compared to that - a lower degree of extensibility and/or elasticity and, consequently, a lower retraction force in the crotch region of the diaper provide an especially high wearer's comfort.

Side panels 10 according to the present invention can also be used in disposable absorbent articles 100 other than diapers where the person skilled in the art can arrange the side panels depending on the construction of such disposable absorbent articles 100 to provide for an improved and/or optimum wearer's comfort.

The extensible side panel 10 may comprise any suitable extensible or stretchable material 22, 22'. The material may be inelastically or elastically extensible whereby the term elastically extensible as used above and below means that the material extends at least in one direction when a force is applied and returns approximately to its original dimensions when the force is removed. The degree of elasticity of an extensible material 22 in a given direction and for a given elongation of, for example, 100 % can be defined as the ratio of
*dimension of material 22 after stretching to 100 % and subsequent relaxation original dimension of material before stretching*

For an essentially elastically extensible material 22 such ratio is usually between 1 and 2 at an elongation of 100% whereas for inelastically extensible materials 22 such ratio can be distinctly greater than 2 which results from the permanent set following deformation and relaxation.

In the present invention extensible materials 22, 22' are preferred where such ratio is for a moderate elongation of, for example, between 50 % and 150 % not more than 1.8, more preferably not more than 1.6 and even more preferably less than about 1.5.

In one embodiment of the present invention, a web 200 is provided comprising an extensible material 22 having a direction of extensibility essentially in the cross-direction. This means that the extensible material 22 can be extended in CD when applying a force essentially in CD; more preferably, the web 200 comprising the extensible material 22 can be stretched by at least 25 %, more preferably by at least 50 % and especially preferably by at least 100 % when applying a force of 4 N/m essentially in CD. The term "essentially in CD" means that the direction of the force deviates from the CD by less than 15°, preferably by less than 10° and especially preferably by less than 5°.

In another embodiment of the present invention a web 200 is provided comprising an extensible material 22 having a direction of extensibility essentially in machine-direction. Stranded elastic materials which are MD stretchable are disclosed, for example, in US 5,681,302, US 4,552,795 and US 3,575,782.

The extensible material 22, 22' can broadly include any material which is capable of being formed or included into a film or laminate layer, and which is extensible or, more preferably, exhibits elastic properties at ambient conditions.

The extensible material 22, 22' may include materials selected from a group comprising essentially isotropic or essentially anisotropic elastic materials. Useful elastic materials preferably exhibit an elongation at break as measured according to ASTM D 882 in the preferred direction of stretchability of at least 50 % or more and, more preferably, of more than 100 %.

Preferred essentially isotropic elastic materials include polyurethane materials and natural or synthetic rubber materials such as, for example, ethylene-propylene diene copolymers (EPDM), and AB- or ABA-type block copolymers such as styrene-butadiene-styrene block copolymers (SBS) or styrene-(ethylene-butylene)-styrene block copolymers (SEBS). Elastic materials of the A-B or A-B-A block copolymer type which are useful in the present invention include, for example, those described in US 3,265,765, US 3,562,356, US 3,700,633, US 4,116,917 and US 4,156,673. Preferred elastic materials of this type are commercially available from Exxon Mobil Corp. under the trade name Vector and from Kraton Polymers Comp. under the trademark Kraton.

Other elastic materials which may be used in the extensible material 22, 22' include elastic polyamide materials and elastic polyester materials. Blends of these elastic materials with each other or with modifying non-elastic materials may also be useful. For example, up to 50 wt.%, but preferably less than 30 wt.% with respect to the mass of the elastic material can be added as stiffening aids such as polyvinylstyrenes, polystyrenes, epoxies, polyolefins or coumarone-indene resins.

These stiffening agents tend to improve the flexibility of the elastic materials. The elastic material may also be blended with viscosity reducing polymers, plasticizers or tackifying resins whereby the latter may be useful to increase the bonding between an elastic layer and further layers in a laminate construction. Short fibres or microfibres can be used to reinforce the elastomeric layer. Glass bubbles or foaming agents may be used to provide elastomeric foam structures. Further additives include, for example, dyes, pigments, antioxidants, antistatic agents, bonding aids, anti-blocking agents, slip agents, heat stabilizers or photostabilizers.

Essentially anisotropic elastic materials or blends including such materials may also be used in the extensible material 22, 22' of the present invention to increase its processability. Anisotropically elastic materials are disclosed, for example, in US 5,885,908, WO 99/51,666, US 5,344,691, US 5,501,679 and US 5,354,597.

The extensible material 22, 22' preferably includes extensible, and, in particular, elastic films, non-wovens, scrims or filament composite materials such as, for example, elastic scrims, elastic laminates comprising one or more exposed woven or non-woven layers in a continuous or intermitted bonding pattern, zero-strain stretch elastic laminates, pre-strained stretch elastic laminates or breathable elastic laminates. The extensible and, in particular, elastically extensible laminates may comprise at least one essentially elastic layer and one or more further less elastic or inelastic layers which can be co-extruded and may comprise, for example, one or more polyolefins, polyamides, polyesters, polyvinyldiene fluoride, polyacrylate or blends thereof. A laminate may also have one or more exposed woven or non-woven layers.

The one or more less elastic or inelastic layers, respectively, tend to temporarily stabilize the web 200 comprising a web of an extensible material 22, 22', in particular, in MD in order to improve the processability of such web 200. The inelastic or less elastic layers are subsequently subjected to an activation step to render the corresponding laminate extensible and preferably elastically extensible.

Preferred activation treatments include for example, MD or CD stretching, ring rolling, embossing, thermoforming, high-pressure hydraulic forming or casting. WO 91715,365 discloses, for example, a micro-textured elastic laminate comprising at least one elastic core layer capable of elastic elongation and at least one co-extruded outer skin layer which is capable of forming a micro-structured surface when said laminate is stretched past the elastic deformation limit of at least one of the skin layers. WO 91/15,364 discloses spatially modified elastic laminates comprising at least one elastomeric layer and at least one thin skin layer. The laminate may be inelastic as formed but is activated in preferential activation zones, for example, by post-laminate-formation stress treatment. Post-laminate-formation stress treatment can be effected by mechanical ablation, scoring, cutting out material, indentation or controlled localized stretching using techniques such as, for example, incremental stretching or ring rolling. The laminate may also be elastic as formed but is rendered partially inelastic, for example, by post-laminate-formation modulus treatment. The post-lamination-formation modulus treatment can include post-formation annealing, selective cross-linking or selective plasticization.

US 6,159,584 discloses a co-extruded elastic film comprising at least one elastic layer and at least one second layer on at least a first face of the elastic layer with at least one face of the co-extruded elastic film being attached to at least a partially extensible non-woven layer.

Breathable elastic laminates are disclosed, for example, in EP 0,784,461. Zero-strain stretch laminates are disclosed, for example, in US 5,156,793, US 5,167,897 or US 5,518,801.

The web 200 may comprise in addition to a web 230 of an extensible material 22, 22' one or more further materials and components such as, for example, a continuous web of a support layer 17 or a discontinuous series of strips of a support layer 17, respectively. The web may further comprise a continuous web or a discontinuous series 216, respectively, of a fastening member 16. Any of such webs may comprise further materials and components such as stiffening materials, fingerlifts, coloured films, printings or registration marks.

The support layer 17, if present, may be selected from a variety of films or sheetings including single- or multilayered films, co-extruded films, laminated films or films comprising foam layers. The layer(s) of such support layers or sheetings 17 may comprise various materials such as, for example, polypropylene, polyvinylchloride, polyethylene terephthalate, polyethylene, polyolefin copolymers or blends of polyolefins such as, for example, a blend of polypropylene, LPDE (low density polyethylene) and/or LLDPE (linear low density polyethylene), non-woven and foamed materials. The thickness of such support layer 17 preferably is between 1-5 and 500 µm and more preferably between 20 and 150 µm. The support layer preferably has a Gurley stiffness value both in CD and MD of the web 200 as evaluated according to TAPPI Standard Test T 543 om-94, of less than about 1,000 milligrams (mg). The Gurley stiffness both in CD and MD preferably is less than 500 mg and especially preferably less than 200 mg.

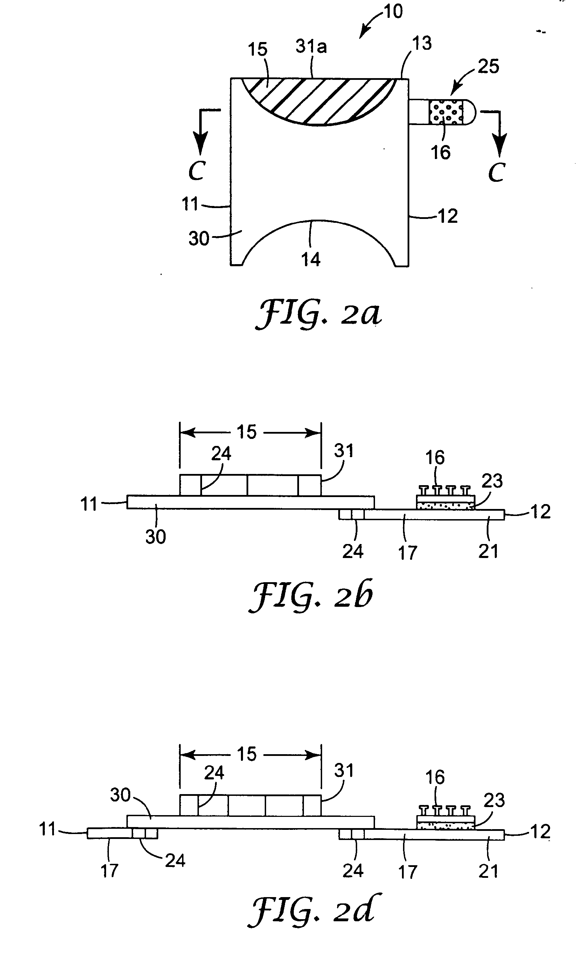

The web 200 may comprise a web 217 of a support layer 17 extending continuously across the full width of the web 230 of an extensible material 22, 22' in the CD of the web 200 but it is preferred that the web 217 of the support layer 17 is present only in certain regions of the web 230 in order not to adversely affect the overall extensibility of the extensible material 22, 22'. In the embodiment of the side panel 10 of Fig. 2a, a fastening tape tab 25 is attached to the second (right) longitudinal edge of layer 30 comprising an extensible material 22, 22'. The fastening tape tab 25 comprises a strip of a support layer 17 bearing a fastening member 16. The strip of a support layer 17 is attached to the first (lower) layer 30 of the extensible material 22, 22', for example, by adhesive means such as pressure-sensitive adhesive means and/or hot-melt adhesive means, ultrasonic bonding, thermal bonding, mechanical bonding, stitching or any combination of these bonding methods. In the embodiment of the side panel 10 of Fig. 2c a continuous support layer 17 is attached to the first (left) longitudinal edge of the first (lower) layer 30 comprising an extensible material 22, 22' to provide the longitudinal edge 11 of the side panel 10 in order to render the side panel 10 more readily attachable, for example, to a disposable absorbent article 100. Another support layer 17 is attached to the right longitudinal edge of the first (lower) layer 30 comprising an extensible material 22, 22' to carry the fastening member 16 and to provide for a fingerlift 21. In the embodiment of the side panel 10 of Fig. 2c the fastening member 16 is attached to the first (lower) layer 30 comprising an extensible material 22, 22' by using a support layer 17 but is also possible that the fastening member 16 is directly applied to the first (lower) layer 30 comprising an extensible material 22, 22' without an additional support layer.

The support layers 17 suitable for use in the extensible side panels 10 of the present invention and the specific constructions referred to above are to illustrate the invention but are not intended to be limiting in any way.

Stiffening materials which may be included, for example, in the extensible side panels 10, the support layer 17 and/or the fastening member 16 include, for example, thermally or sonically structured surfaces or additional layers.

The fastening member 16 may be provided by interlocking, mechanical-type fasteners, by adhesive fastening means and/or by other fastening means. To provide a releasable and refastenable mechanical closure system, for example, in a disposable absorbent article, such article may comprise in addition to the fastening member 16 a corresponding complementary landing zone 108 as is indicated in Figs. 3a and 3b.

A suitable mechanical closure system may comprise two interlocking fastening members, one of them being a hook (or male) fastening member and the other being a loop (or female) fastening member. The fastening member 16 applied to the extensible side panel 10 may comprise the hook fastening member or the loop fastening member, respectively, but preferably comprises the hook fastening member. The hook fastening member usually comprises fastening elements comprising a stem supported by a backing and an enlarged section which is positioned at the end of the stem opposite to the backing from which the stem emanates. The stems may have any cross-section such as, for example, essentially circular, square, rectangular or cross-shaped, and the enlarged sections may have any shape such as hooks, T's, J's, mushroom-type heads (including concavely curved heads and disc-shaped heads) or any other shape allowing for engagement with complementary female fastening elements. The male fastening elements can also be formed by stems having no enlarged section at the end of the stem whereby such stems preferably are essentially cylindrical, conical or pyramidal. The male fastening elements preferably emanate from and are integral with a backing through which the fastening means 16 may be attached, for example, to the support layer 17 or to the first (lower) layer 30 of the extensible material 22. It is, however, also possible that the male fastening elements are attached individually or in patches, for example, to the support layer 10 to the first (lower) layer 30 of the extensible material 22, 22'. Male fastening elements and their method of manufacturing are disclosed, for example, in US 5,077,870, US 5,679,302, US 4,894,060, US 4,984,339 and US 5,781,969.

The extensible side panels of the present invention can preferably be obtained both from webs 200 having a direction of extensibility in CD and from webs 200 having a direction of extensibility in MD as is explained in more detail below.

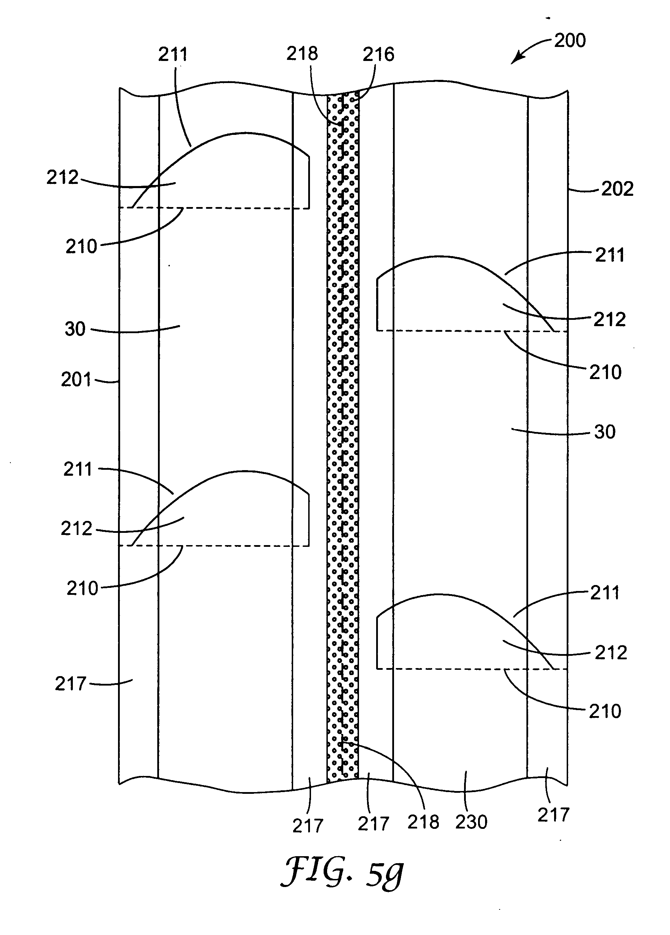

In a preferred method of manufacturing the side panels 10 of the present invention a web 200 comprising a web of an extensible material 22, 22' having a direction of extensibility essentially in CD, is provided in MD as is illustrated, for example, in Figs. 5a - 5m. The width of the web 200 may essentially correspond to the width of the side panel in the lateral direction as is illustrated, for example, in Figs. 5a and 5d. In a more preferred embodiment the width of the web 200 comprising an extensible material 22 having a direction of extensibility in the CD, may essentially correspond to the width of two, four, six, ... 2n side panels in CD as is illustrated for two side panels, for example, in Figs. 5g and 5j. In this case the left part of the web 200 provides, on applying cuts along the uncut portions of the designated fold lines 210 and the cut line 219 through the web 216 of the fastening means 16 (see Fig. 5i), a sequence of a pair of side panels which can be applied, for example, to the right and left side of a diaper.

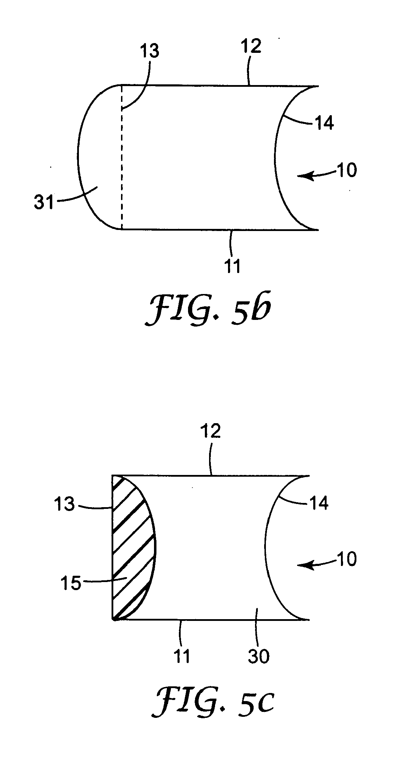

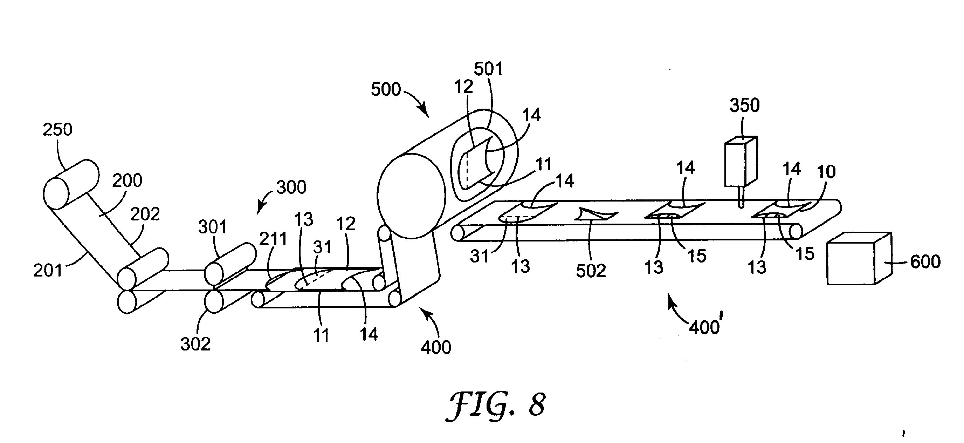

In the next step a series of intermediate cut lines 211 is applied to the web 200 as is illustrated, for example, in Figs. 5a and 5d. In the method illustrated, for example, in Fig. 5a, the intermediate cut lines 211 extend over the full width of the web 200 in CD so that a series of individual precursors of the extensible side panel 10 is obtained. The second (upper) layer 31 is folded around the designated first top lateral edge 13 (shown in broken lines in Fig. 5a) and secured to the first (lower) layer 30 to provide the side panel 10 shown in Fig. 5c. It was found by the present inventors that the second (upper) layer 31 can be folded more precisely around the designated first top lateral edge 13 if such folding operation is carried out in CD. Therefore, the precursor of the extensible side panel 10 is preferably rotated counter-clockwise by 90° prior to the folding step (see Figs. 5b and 8).

In the method illustrated, for example, in Figs. 5d or 5g, the intermediate cut lines 211 do not extend over the full width of the web 200 in CD so that foldable flaps 212 are obtained which are adjacent to the designated fold lines 210. In the embodiment of Fig. 5d, the integrity of the web is thus maintained when applying the intermediate cut lines 211. The intermediate cut line 211 introduced in Fig. 5d extends to the designated fold line 210 at two or more points so that the resulting one or more foldable flaps 212 can be folded over in the next step along said designated fold line 210.

The intermediate cut lines 211 can generally be applied, for example, by means of a rotating knife, by laser cutting, die cutting or by other cutting means. Depending on the direction of extensibility of the web of extensible material 22 comprised in web 200 and the specific construction selected, the series of intermediate cut lines 211 is applied essentially in MD (see, for example, Figs. 5a or 5d) or both in CD and MD (see, for example, Figs. 5g and 6a).

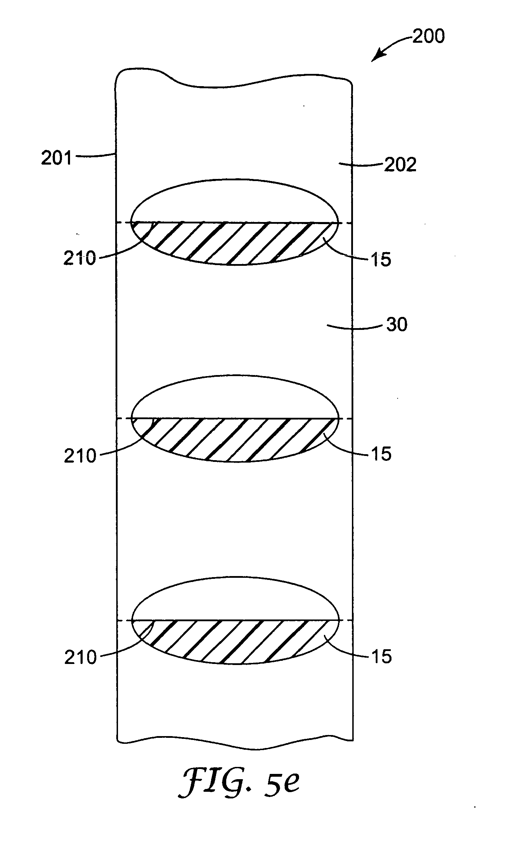

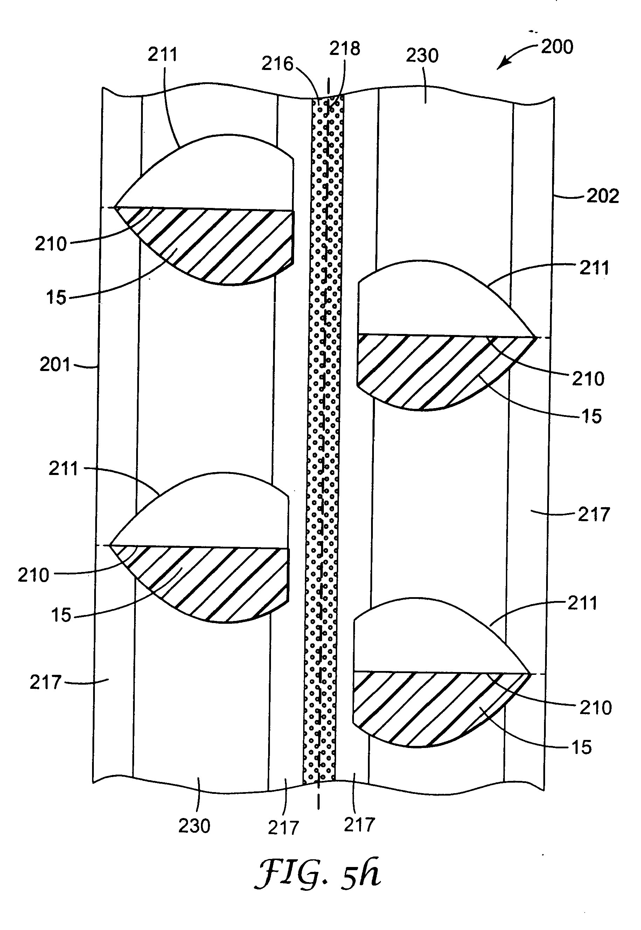

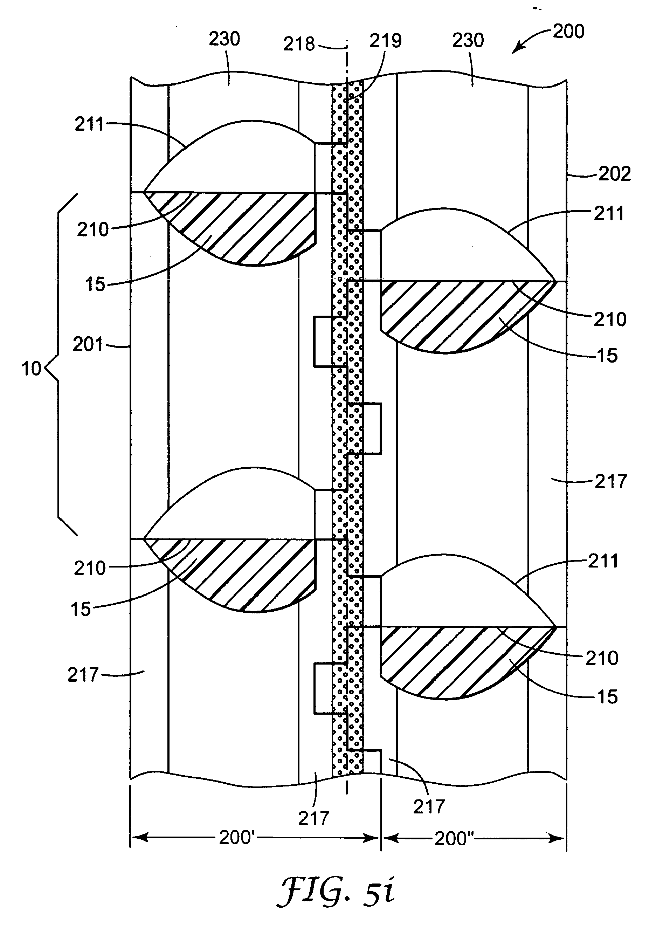

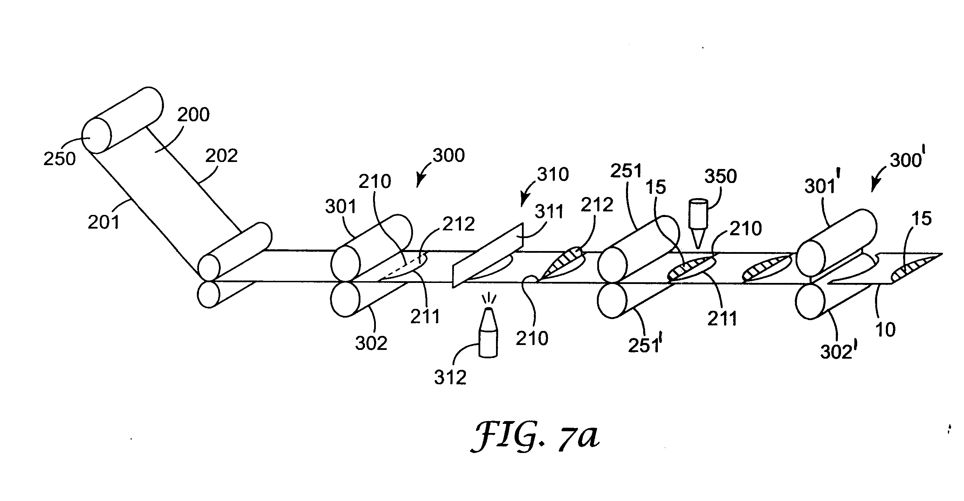

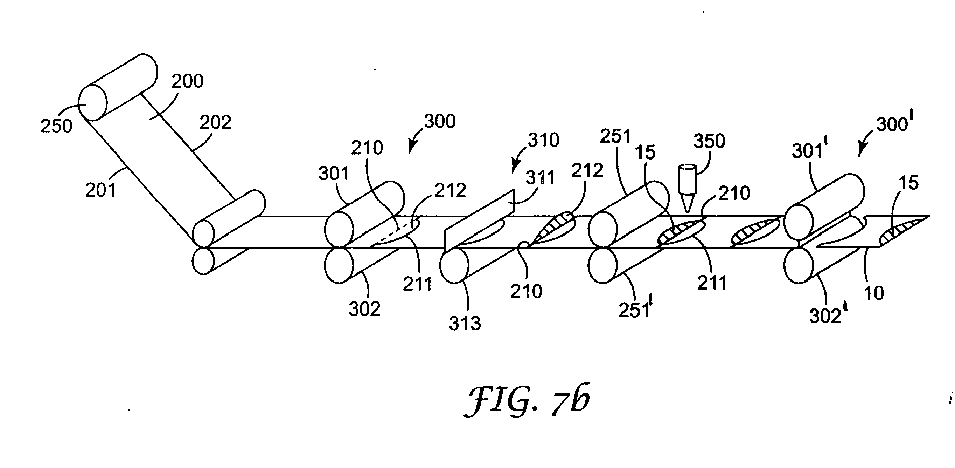

In the next step of the method illustrated, for example, in Figs. 5e or 5h using, for example, the equipment illustrated in Figs. 7a and 7b, the foldable flaps 212 are folded over. This can be accomplished, for example, by passing the web 200 over a sprocket wheel 313 (see Fig. 7b) to lift up the flap 212. Alternatively, the flap 212 can be lifted up by blowing pressurized air onto the bottom side of the web via the nozzle 312 (see Fig. 7a), or by using other mechanical or pneumatic lifting means. The lifted foldable flap 212 is then passed, for example, through a nip provided by the rollers 251, 251' to complete the folding-over step and press the foldable flap 212 onto the web 200. The foldable flap 212 is then attached to the web 200 by a sealing unit 350 using any bonding method including, for example, adhesive bonding, thermal bonding, ultrasonic bonding and/or mechanical attachment. The flap 212 is preferably bonded to the underlying web 200 in a non-continuous, intermittent bonding pattern in order to maintain the extensibility of the extensibly reinforced region 15.

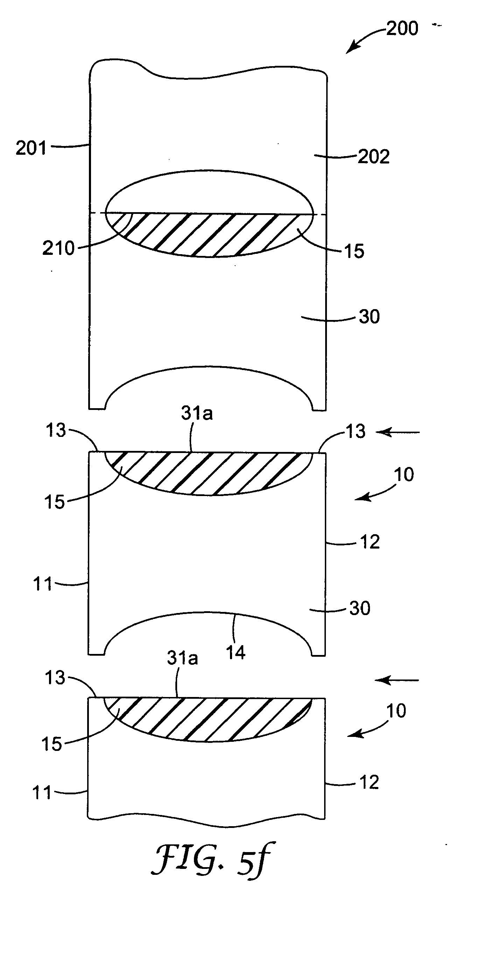

In the final step of the method illustrated, for example, in Figs. 5f or 5i, cut lines are applied by means of, for example, a rotating knife along the designated fold lines 210 to provide individual side panels according to the present invention. In the method of Figs. 5g - 5i an additional cut line 219 needs to be applied through the web 216 of fastening member 16 to provide two separate sub-webs 200', 200".

The side panels 10 of the present invention can also be obtained by using other methods. It is, for example, possible in the methods of Figs. 5d - 5f or 5g - 5i to cut out and discard the foldable flap 212 by cutting along the designated fold lines 210 and omitting the step of folding over such flap. The overlying layer 31 of the reinforced region 15 can be die-cut, for example, from a separate web and applied, for example, by means of a cut and place applicator comprising a vacuum roll and a rotating knife. Such method may be advantageous for the manufacturing of side panels comprising an underlying layer 30 and an overlying layer 31 within the reinforced region 15 which comprise different extensible materials 22, 22'.

If the web 200 comprises a web of a precursor of the extensible material 22, 22' which needs to be activated to become extensible and/or elastic, such activation step can be included, for example, into the methods illustrated in Figs 5d - 5f or 5g - 5i prior to cutting along the designated fold lines 210, i. e. prior to separating the web into individual side panels. Alternatively, the web can be activated in these methods prior to applying the intermediate cut lines 211. Suitable activation techniques include, for example, ring rolling or stretching by means of tenter stretching of disc stretching equipment, respectively. Activation in CD can be advantageously performed, for example, by stretching the web in CD using a tenter method or a pulley method disclosed, for example, in GB 849,436 B. An advantageous CD stretching device is disclosed, for example, in Fig. 7 of US 6,124,001. Stretch activation in MD can be performed, for example, by passing the web over rolls in MD having a differential rotation speed.

In another preferred method of manufacturing the side panels 10 of the present invention, a web of an extensible material 22, 22' having a primary direction of extensibility essentially in MD, is provided in MD as is illustrated, for example, in Fig. 6a.

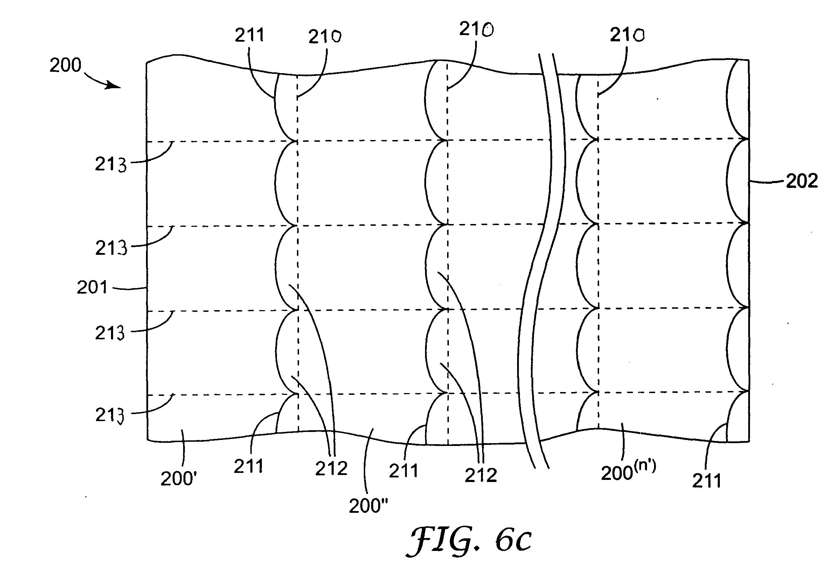

The width of the web 200 in CD is selected to provide n-1 webs of side panels as is illustrated, for example, in Figs. 6a or 6c. The web 200' does not exhibit a reinforced region 15 and is discarded. The cut-out area between the intermediate cut lines 211 and the second (right) longitudinal edge 202 of the web 200 is also discarded.

In the final step of the method illustrated in Figs. 6a and 6b, a series of cut lines is applied along the designated fold lines 210 to provide a series of separate sub-webs 200', 200", ... 200^{(n')}. The individual side panels 10 are obtained from such sub-webs by cutting along the designated cut lines 213.

The methods of manufacturing the extensible side panels 10 of the present invention described above are merely to illustrate the invention without restricting it in any way. The methods of manufacturing illustrated in Figs. 7a, 7b and 8 in conjunction with Figs. 5a - 5m and 6a - 6c are especially advantageous because
- they are specifically suited to be applied under high speed manufacturing conditions, and
- they do essentially not generate any scrap
so that they allow for advantageous manufacturing costs.

### Detailed description of the figures

*Figs. 1a - 1c* schematically show a first embodiment of an extensible side panel 10 of the present invention comprising a first layer 30 comprising an extensible material 22. The side panel 10 is bordered by the first and second longitudinal side edges 11, 12, a first top lateral edge 13 and a second bottom lateral edge 14. The side panel 10 comprises a reinforced region 15 which is arranged adjacent and in parallel to the top lateral edge 30a. The side panel 10 further comprises a second (upper) layer 31 comprising an extensible material 22'. The top lateral edge 31 a of the second layer 31 is arranged adjacent and in parallel to the top lateral edge 30a of the first layer. The top lateral edges 30a, 31 a essentially coincide and form the first top lateral edge 13. This can be seen from Fig. 1 c showing a cross-sectional view of the side panel 10 of Fig. 1a along the line B-B. The second (upper) layer 31 is attached to the first (lower) layer 30 by means of the welded attachment lines 24 to form the extensibly reinforced region 15 as can be taken from the cross-sectional view along the lines A―A and B―B shown ins Figs. 1 band 1 c. The extension of the top lateral edge 31a of the second layer 31 is smaller than the extension of the top lateral edge 30a of the first layer 30 so that the side panel 10 of Fig. 1 a can be manufactured, for example, using the method illustrated in Figs. 5d - 5f in connection with Figs. 7a and 7b. The MD and the CD of the side panel 10 are indicated in Fig. 1a.

*Fig. 1d* schematically shows another embodiment of an extensible side panel 10 of the present invention which is similar to the construction of Fig. 1 a but comprises two reinforced regions 15.

*Fig. 1e* schematically shows another embodiment of an extensible side panel 10 of the present invention which is similar to the construction of Fig. 1 a but comprises a straight second bottom lateral edge 14.

*Fig. 1f* illustrates the hypothetical area 19 which is bordered by the second bottom lateral edge 14 and by a hypothetical cut line 18 which is parallel to the first top lateral edge 13 and extends through the two lowest points of the second bottom lateral edge 14. The embodiment of the side panel of Fig. 1f is similar to the construction of Fig. 1 a but the area of the reinforced region 15 is smaller than the area of the hypothetical region 19. When using, for example, the method of manufacturing which is exemplified in Figs. 5d - 5f below, the area of the reinforced region 15 can be equal to or smaller than the area of the hypothetical region 19.

*Fig. 1g* schematically shows another embodiment of an extensible side panel 10 of the present invention which is similar to the construction of Fig. 1 a but includes an angle between the first longitudinal edge 12 representing the MD and the first top lateral edge 13 which is smaller than 90°.

*Figs. 1h, 1i, 1j, 1k and 1l* show other embodiments of an extensible side panel 10 of the present invention which are similar to the embodiments illustrated in Figs. 1 a, 1 d, 1 e, 1 f, 1 g and 1 h, respectively, but differ from these constructions in that the extension of the top lateral edge 31 a of the second (upper) layer 31 comprising an extensible material 22 is equal to the extension of the first top lateral edge 13 of the side panel 10. The side panels of Figs. 1 h, 1i, 1j, 1 k and 1l can be manufactured, for example, using the method illustrated in Figs. 5a - 5c in connection with Fig. 8.

*Figs.* 2a and 2b schematically show another embodiment of an extensible side panel 10 of the present invention which includes the construction of Fig. 1 a to which a fastening tab 25 comprising a fastening member 16 is attached. The cross-sectional view of Fig. 2b which is taken along line B-B in Fig. 2a, shows that a strip of a support layer 17 (also referred to as the backing of the fastening tab) is attached to the layer 30 next to the second longitudinal edge 12 and beside the reinforced region 15. The strip of a support layer 17 is attached to the first (lower) layer 30 of the extensible material 22 by welded attachment lines 24 such as, for example, ultrasonic bonding lines. The strip of a support layer 17 bears a patch of fastening members 16 which is attached via adhesive layer 23, and provides a fingerlift 21.

*Figs.* 2c *and* 2d schematically show another embodiment of an extensible side panel 10 of the present invention which includes the construction of Fig. 1 h additionally comprising a support layer 17 bearing a fastening member 16 along the second lateral edge 12 and a further support layer 17 along the first lateral edge 11. The cross-sectional view of Fig. 2d which was taken along line D―D in Fig. 2c, shows that support layers 17 are attached along both the first and second longitudinal lines 11, 12 of the first (lower) layer 30 comprising an extensible material 22. Both support layers 17 are secured to the first (lower) layer 30 comprising an extensible material 22 via welded attachment lines 24. The support layer 17 attached along the first longitudinal edge 11 of the first layer 30 facilitates assembly of the side panel 10 to the chassis 110 of an absorbent article. The support layer 17 attached along the second longitudinal edge 12 of the layer 30 bears a fastening means 16 secured via adhesive layer 23, and provides a fingerlift 21.

*Fig.* 2e schematically shows another embodiment of an extensible side panel 10 of the present invention which is similar to the construction of Fig. 2c with the difference that a meandering cut line is applied through the fastening member 16. The meandering cut line provides two separate fastening tabs 25 each comprising a fastening means 16 and a fingerlift 21.

*Figs 2f and* 2g schematically show other embodiments of extensible side panels of the present invention which are similar to the side panels 10 of Fig. 2a and 2e, respectively. The top lateral edge 31a of the second (upper) layer 31 of the side panel of Figs. 2f and 2g, respectively, has a larger extension in comparison to the top lateral edge 31 a of the side panels of Figs. 2a and 2e, respectively.

*Fig.* 3a schematically shows a top view on a diaper 100. The diaper comprises a chassis 110 to which two side panels 10 according to Fig. 2f are attached. The chassis comprises an absorbent core 103 which is sandwiched between the top sheet 101 and the back sheet 102 (not shown in Fig. 3a). The diaper comprises a back waistband region 106 and a front waistband region 107. The side panels 10 are attached via attachment regions 109 along the first and second longitudinal edges 104, 105 of the diaper 100 whereby the side panels extend from the back waistband region 106 to the crotch region. The attachment in attachment regions 109 may include any attachment mechanism including adhesive bonding, thermal bonding, mechanical bonding an/or ultrasonic bonding. The side panels 10 each comprise a fastening tab 25 comprising a male (hook) mechanical fastening member 16. The diaper 100 comprises a landing zone 108 attached to the back sheet 102 in the front waistband region 107. The landing zone comprises a fastening member such as a female (loop) mechanical fastening member which is complementary to the male mechanical fastening member 16 on the fastening tape tab.

Alternatively, if the back sheet 102 comprises a suitable exposed non-woven layer, the male (hook) fastening means on the fastening tab 25 may be engageable with such non-woven layer, and the landing zone 108 may be omitted.

*Fig.* 3b schematically shows a top view on another embodiment of a diaper 100 of the present invention in the direction of its top sheet 101. The diaper of Fig. 3b is similar to that of Fig. 3a but the male (hook) mechanical fastening means are arranged along the second longitudinal edge 12 of the side panel 10. The landing zone 108 is split into two patches which are rearranged correspondingly to allow for an interaction between the fastening member 16 and such patches of the landing zone 108.

*Fig.* 4 shows a perspective view of a training pant 100 according to the present invention. The training pant comprises a chassis 110 and two side panels 10 according to Fig. 1 h. Each side panel is attached along both of its longitudinal edges 11, 12 via attachment regions 109 to the corresponding longitudinal edges 104, 105 of the diaper so that a pull-on construction is obtained. The attachment in the attachment regions 109 may include any attachment mechanism including adhesive bonding, thermal bonding, mechanical bonding and/or ultrasonic bonding.

*Figs.* 5a - 5c schematically illustrate a preferred method of manufacturing side panels 10 according to Fig. 1 h above.

*In Fig.* 5a, a continuous web 200 comprising an extensible material 22 is provided in the MD whereby the web 200 exhibits a direction of extensibility or elasticity respectively, in the CD. The MD and the CD of the web are indicated in Fig. 5a.

The web 200 has longitudinal edges 201, 202 in the MD which correspond to the first and second longitudinal edges 11, 12, respectively, of the side panel 10. The web exhibits designated first top lateral edges 13 around which the foldable flaps 212 defined by the designated intermediate cut lines 211 and said designated first top lateral edges 13, are folded in a later process step (see Fig. 5c). The designated intermediate cut lines 211 and the designated first top lateral edges 13 are therefore shown in the running integral web 200 (upper part of Fig. 5a) in broken lines only.

A series of intermediate cut lines 211 (shown in solid lines) is made into the web. The intermediate cut lines 211 extend over the full width of the web 200 in CD and intersect its longitudinal edges 201, 202 so that a series of individual precursors of the side panel 10 of the present invention is obtained. Such precursors exhibit first and second longitudinal edges 11, 12, a second bottom lateral edge 14 and a designated first top lateral edge 13 (shown in broken lines). The area between the designated first top lateral edge 13 and the intermediate cut line 211 forms the foldable flap 212 which corresponds to the second (upper) layer 31.

It was found by the present inventors that the foldable flap 212 can be folded around the designated first top lateral edge 13 more precisely and at higher speeds in a continuous manufacturing process if such folding is performed in CD. Therefore the precursor of the side panel 10 is rotated counter-clockwise by 90° in Fig. 5b before the foldable flap 212 (corresponding to the second (upper) layer 31) is folded over around the designated first top lateral edge 13 and is attached to the first (lower) layer 30 comprising an extensible material 22 to provide the reinforced region 15 in Fig. 5c. The folding step exposes the designated first top lateral edge 13 to become the first top lateral edge 13 of the side panel 10.

*Figs. 5d -* 5f schematically illustrate a preferred method of manufacturing side panels 10 according to Fig. 1 a above.

*In Fig.* 5d, a continuous web 200 of an extensible material 22 is provided in MD. The web has longitudinal edges 201, 202 in MD which correspond to the first and second longitudinal edges, respectively, of the side panel 10. The web exhibits designated fold lines 210 along which the foldable flap 212 is folded in a later process step (see Fig. 5e). The designated fold lines 210 are therefore indicated in Fig. 5d as broken lines only. A series of intermediate cut lines 211 is made into the web which do not extend over the full width of the web 200 in CD so that the integrity of the web 200 is maintained during this process step. The intermediate cut lines 211 are shown in full lines to indicate that such cuts are actually made in the process step shown in Fig. 5d. For the designated side panel 10 preceding an intermediate cut line 211, such intermediate cut line 211 corresponds to the second bottom lateral edge 14 of such side panel 10. For the designated side panel 10 following in MD the area between the intermediate cut line 211 and the designated fold line 210 corresponds to the foldable flap 212 which - when folded over and attached to the underlying web - forms the second (upper) layer 31.

*In Fig.* 5e, the foldable flaps 212 were folded over to provide reinforced regions 15. The flap 212 which was folded over provides the overlying layer 31 of the reinforced region 15. The portion of the web 200 onto which the flap is folded over, represents the underlying layer 30 of the reinforced region 15. The overlying layer 31 is attached to the underlying layer 30 by any bonding mechanism including adhesive bonding, thermal bonding, mechanical bonding and/or ultrasonic bonding whereby intermittent bonding between the first (lower) and second (upper) layers 30, 31 is preferred. In the embodiment of Fig. 5e the area of the reinforced region 15 corresponds to the area between the intermediate cut line 211 and the designated fold line 210 which is also referred to as hypothetical region 19.

*In Fig. 5f,* the web 200 is cut in CD in the direction of the designated fold lines 210 thereby releasing individual side panels 10.

*Figs.* 5g - 5i illustrate another preferred method of manufacturing side panels 10 according to the invention which provides side panels according to Fig. 2e above.

*In Fig.* 5g a web 200 is provided in MD comprising from left to right a web 217 of a support layer 17, a first web 230 comprising an extensible material 22, a second web 217 of a support layer 17 bearing a web 216 of a fastening member 16 which is attached to such web of support layer 17 by any method, a second web 230 comprising an extensible material 22 and a third web 217 of a support layer 17. The web exhibits a longitudinal centre line 218 extending through the web of the fastening member 216. A series of intermediate cut lines 211 is made in MD into the two parts of the web 200 separated by the centre line 218. Each intermediate cut line 211 starts at some point of the corresponding designated fold line 210 (shown as a broken line) within the web 217 of a support layer 17, i. e. at some distance from the corresponding longitudinal edge 201, 202, extends through the corresponding webs 217 and 230 and returns to the designated fold line 210 with a section extending in MD; this section corresponds to a portion of the meandering cut line 219 applied around the centre line in the step illustrated in Fig. 5i below. The integrity of the web 200 is maintained in the step illustrated in Fig. 5g.

*In Fig.* 5h the foldable flaps 212 bordered by the intermediate cut line 211 and the designated fold line 210 have been folded over to provide reinforced regions 15 as was described in connection with Fig. 5e above.

*In Fig.* 5i a meandering periodic cut line 219 is applied around the centre line 218 thereby separating the web 200 into two sub-webs 200', 200". The meandering cut line 219 provides two fastening tabs 25 per designated extensible side panel 10 whereby each fastening tab comprises a fastening member 16 and a fingerlift 21; this means in other words that the meandering cut line 219 has a periodicity which corresponds to half the distance between two designated fold lines 210.

Then, the two sub-webs 200', 200" are cut in the direction of the designated fold lines 210 to release individual side panels 10.

The method illustrated in Figs. 5g - 5i thus provides a sequence of a pair of "right" and "left" side panels. The right side panels are provided by cutting the left sub-web 200' along the designated fold lines 210. Likewise, the left side panels are provided by cutting the right sub-web 200" along the designated fold lines 210.

*Figs. 5j -* 5m illustrate another preferred method of manufacturing side panels according to the invention which provides side panels 10 according to Fig. 2g above.

In the first step shown in Fig. 5j, a series of pairs of precursors of side panels is obtained by applying
- a series of intermediate cut lines 211 starting at the intersection point between the corresponding longitudinal edges 201, 202 of the web and the corresponding designated first top lateral edge 13 (shown as a broken line), extending through the corresponding webs 217 and 230 and returning to the designated first top lateral edge 13 with a section extending in MD (whereby this section corresponds to a portion of the meandering cut line 219),
- a meandering cut line 219 around the centre line 218.

The precursors of the side panel 10 each comprise a foldable flap 212 formed by the corresponding intermediate cut line 211, and the designated first top lateral edge 13 when folded over around the designated first top lateral edge 13 the foldable flap 212 corresponds to the second (upper) layer 31 comprising an extensible material 22.

*Fig. 5k* shows that the left precursor of a pair of side panels 10 is then rotated counter-clockwise by 90° while the right precursor of such pair is then rotated clockwise by 90° in order to facilitate the subsequent folding step illustrated in Fig. 51 and 5m. It was found by the present inventors that the foldable flap 212 can be folded around the designated first top lateral edge 13 more precisely and at higher speeds in a continuous manufacturing process if such folding is performed in CD.

*Figs.* 6a - 6b schematically illustrate a preferred method of manufacturing side panels according to Fig. 1 a above whereby the web of the extensible material 22 provided in MD, exhibits a direction of extensibility or elasticity, respectively, in MD.

The web 200 is conceptionally divided by n longitudinal designated fold lines 210 in n sub-webs 200', 200", ... 200^{(n')}. No cuts are applied in the direction of the longitudinal designated fold lines 210 in this step so that they are shown in broken lines. A series of intermediate cut lines 211 is applied to each sub-web 200', 200", ... 200^{(n')} along the designated fold lines 210 in MD thereby creating foldable flaps 212 which can be folded over in CD around the designated fold lines 210 as was described above. The intermediate cut lines 211 applied in MD for each sub-web are separated from each other in MD by a certain distance so that the integrity of the web 200 is maintained in this step.

*In Fig.* 6b, the foldable flaps 212 are folded over around the longitudinal designated fold lines 210 to provide reinforced regions 15 as was described above, for example, in connection with Fig. 5e.

Cutting the web along the longitudinal designated fold lines 210 provides n sub-webs 200', 200", ... 200^{(n')}. The left sub-web designated as sub-web 200' is discarded because it does not comprise the reinforced region 15. The remaining n-1 sub-webs are cut along the designated cut lines 213 to release individual side panels 10 as was described above.

*Fig.* 7a schematically illustrates a method of manufacturing individualized extensible side panels 10 of the type shown, for example, in Fig. 1 a using a web of an extensible material having a direction of extensibility in CD. Fig. 7a also schematically shows an apparatus useful for carrying out said method. The web 200 comprising an extensible material 22 having a direction of extensibility in CD is unwound from the supply roll 250 and passed by the rotary die cut unit 300 comprising the cut roller 301 and the pressure roller 302, to introduce a series of intermediate cut lines 211 into the web 200. The intermediate cut lines 211 do not extend over the full width of the web 200 in CD so that the integrity of the web 200 is maintained in this cutting step.

The foldable flaps 212 which are defined by the intermediate cut lines 211 and the designated fold lines 210, are pushed up in the folding device 310 and pressed down onto the web 200 by passing the web 200 through the nip provided by a pair of rollers 251, 251'. The folding device comprises a folding bracket 311 and a nozzle 312 supplying pressurized air. The folding bracket is, for example, a thin sheet metal contacting the web 200 along the designated fold line 210 and keeping the web 200 in place while pressurized air is blown by the nozzle 312 from beneath against the foldable flap 212. This ensures that the foldable flap 212 is pushed up and folded by about 90° precisely around the designated fold line 210. Folding over is completed by passing the web comprising partially pushed-up foldable flaps 212 through the nip provided by the rollers 251, 251'.

The foldable flap 212 is secured to the underlying layer 30 on the web in a sealing unit 350 which preferably provides an intermittent bonding pattern between the second (upper) layer 31 (which corresponds to the foldable flap 212) and the underlying portion of the web (which corresponds to the first (lower) layer 30)). The sealing unit may comprise, for example, an ultrasonic bonding unit but any other bonding method including adhesive bonding, thermal bonding or mechanical bonding may be used as well. In a second cutting unit 300' comprising another cut roller 301' and another pressure roller 302', a series of cuts is applied to the web along the designated fold lines 210 thereby releasing individual side panels 10 of the type shown in Fig. 1 a.

The method of manufacturing and the corresponding equipment illustrated in Fig. 7a are conceptionally explained also in Figs. 5d - 5f.

*Fig.* 7b schematically illustrates a method of manufacturing side panels 10 of the type shown in Fig. 1 a and a corresponding suitable equipment which is identical with the method and the equipment shown in Fig. 7a with the exception that a sprocket wheel 313 is used in the folding device 310 instead of the nozzle 312 providing pressurized air in order to push up the foldable flaps 212. The method and apparatus of Fig. 7b employ a web of an extensible material 22 having a direction of extensibility in CD.

*Fig.* 8 schematically illustrates a method of manufacturing individualized foldable side panels 10 of the type shown, for example, in Fig. 1h using a web of an extensible material having a direction of extensibility in CD. Fig. 8 also schematically shows equipment useful for carrying out said method.

In Fig. 8, the web 200 comprising an extensible material 22 having a direction of extensibility in CD is unwound from the supply roll 250 and fed into the rotary die cut unit 300 comprising the cut roller 301 and the pressure roller 302. The rotary die cut unit 300 introduces a series of intermediate cut lines 211 into the web extending over the full width of the web 200 in CD so that a series of individual precursors of the side panel 10 is obtained which are picked up by the vacuum belt 400.

The vacuum belt 400 transfers such precursors onto the turning device 501 of the vacuum wheel 500. The turning device 501 rotates said precursors counter-clockwise by 90° and releases the turned precursors to a second vacuum belt 400'. The second vacuum belt passes the precursors by a folding bracket 502 which folds the foldable flap 212 defined by the intermediate cut line 211 and the designated first top lateral edge 13, precisely around said first top lateral edge 13 and pushes it down onto the precursor. The foldable flap 212 corresponds to the second (upper) layer 31, and the portion of the precursor it is folded onto is the first (lower) layer 30.

The second (upper) layer 31 is secured to the first (lower) layer 30 preferably intermittently by the sealing unit 350 as was described above for Fig. 7a, to provide individual side panels 10 of the type of Fig. 1 h. The side panels 10 may be stored in the storage container 600 or alternatively be applied to a disposable absorbent article 100 in a continuous manufacturing line.

The method of manufacturing and the corresponding equipment illustrated in Fig. 8, are also explained conceptionally in Figs. 5a - 5c above.

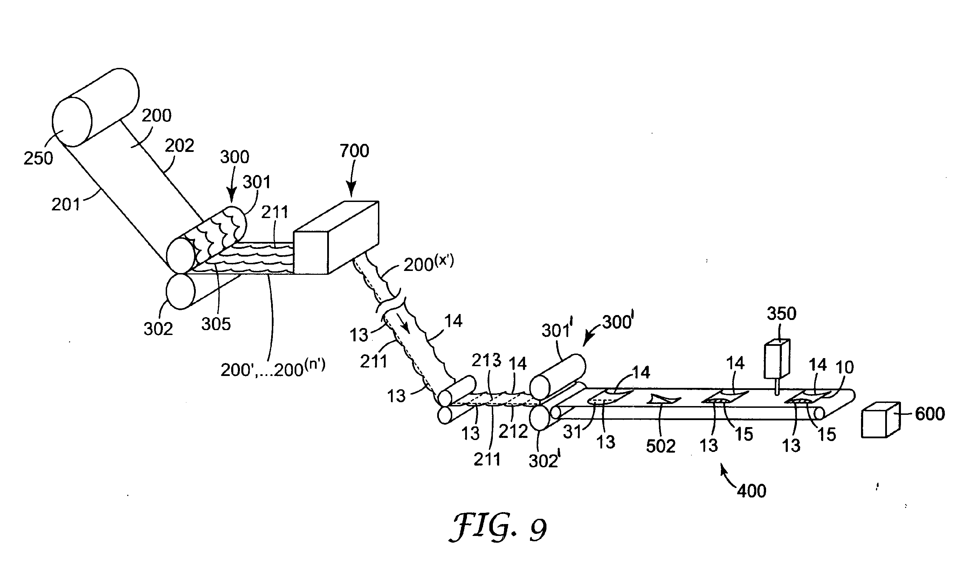

*Fig.* 9 schematically illustrates a method of manufacturing individualized foldable side panels 10 of the type shown, for example, in Fig. 1 h using a web 200 of an extensible material 22 having a direction of extensibility in MD. Fig. 9 also schematically shows an apparatus useful for carrying out said method. The web 200 comprising an extensible material 22 having a direction of extensibility in MD is unwound from the supply roll 250 and passed by a rotary die cut unit 300 comprising a cut roller 301 and a pressure roller 302. The cut roller comprises a series of sinusoidally shaped, essentially parallel circumferential knifes 305, to introduce a series of n' intermediate cut lines 211 into the web 200 extending continuously along the web 200 in MD as is illustrated in Fig. 6c above. The series of n' intermediate cut lines 211 provides for n' individual sub-webs 200', 200", ... 200 ^{(n')}, which were separated from each other in a separation unit 700 for further processing. Fig. 9 illustrates further processing of the sub-web 200^{(x')} with 1 ≤ x ≤ n.

The sub-web 200^{(x')} is then fed into another rotary die cut unit 300' comprising a cut roller 301' and a pressure roller 302'. The rotary die cut unit 300' introduces a series of designated cut lines 213 into the sub-web 200^{(x')} extending over the full width of the web 200 in CD so that a series of individual precursors of the side panel 10 is obtained which are fed onto the vacuum belt 400.

The vacuum belt 400 passes the precursors by a folding bracket 502 which folds the foldable flap 212 defined by the intermediate cut line 211 and the designated first top lateral edge 13, precisely around said first top lateral edge 13 and pushes it down onto the precursor. The foldable flap 212 corresponds to the second (upper) layer 31, and the portion of the precursor it is folded onto is the first (lower) layer 30.

The second (upper) layer 31 is secured to the first (lower) layer 30 preferably intermittently by the sealing unit 350 as was described above for Fig. 7a, to provide individual side panels 10 of the type of Fig. 1 h. The side panels 10 may be stored in the storage container 600 or alternatively be applied to a disposable absorbent article 100 in a continuous manufacturing line.

The method of manufacturing and the corresponding equipment illustrated in Fig. 9, are also explained conceptionally in Figs. 6a - 6c above.

### Examples

### Materials used in the examples

- BR 2000 elastic laminate which is commercially available from 3M Company, St. Paul, MN, USA. This elastic laminate comprises an elastic multilayer film comprising an SIS (styrene-isoprene-styrene) based elastic core layer bearing on both sides a polypropylene skin layer. The multilayer film is stretched in CD to a multiple of its original width. Non-woven layers (polyester spunbond non-woven, basis weight of about 20 g/m²) are adhesion-bonded to both sides of the multi-layer film while it is maintained in its stretched state. The resulting elastic laminate is subsequently relaxed.
- COBAN™ elastic laminate which is commercially available from 3M Company, St. Paul, MN, USA. This elastic laminate comprises an elastic film comprising an elastic filament material with a basis weight of about 6-8 g/m² with a CD density of 10 strands per inch (25,4 mm). The elastic film is stretched in MD to a multiple of its original length. Non-woven layers (polyester spunbond non-woven, basis weight of about 10 - 20 g/m²) are adhesion-bonded to both sides of the elastic film while it is maintained in its stretched state. The resulting elastic laminate is subsequently relaxed.

### Test methods

### Stress-strain curves (hysteresis tes)

The tests were carried out according to DIN 53835. A 25 mm x 65 mm sample is cut from the material to be tested so that the direction of the 65 mm side corresponds to the direction of the first top lateral edge 13 of the side panel 10, the direction of the top lateral edge 30a of the first layer 30 and/or to the cross-direction CD of the web 200 or of the side panel 10, respectively, i. e. to a direction of extensibility. The sample is mounted in a tensile testing machine commercially available from Zwick GmbH & Co. KG, Ulm, Germany, under the trade designation Zwick Roell 1010 so that it can be stretched in the direction of extensibility. The upper and lower jaws of the tensile tester were 25 mm apart; the sample was centered in the tensile tester between the jaws so that 20 mm of the sample were gripped on each side by the jaws while 25 mm of the sample were held between the jaws. The jaws had a height of 30 mm (in the direction normal to the plane of the sample) in order to minimize slip and breakage in the jaws. The jaws were then separated at a rate of 500 mm/min (first upload) for 25 mm (100 % elongation). The jaws were held at an elongation of 100 % for 1 second and then returned to the zero elongation position (first download). Then the jaws were returned to the 100 % elongation position at a rate of 500 mm/min (second upload). The jaws were again held at an elongation of 100 % for 1 second after which they were returned to the zero elongation position to conclude the test (second download). In all cases stress values were recorded in N/inch as a function of the elongation in % stretch.

### Example 1

Extensible side panels 10 of the type of Fig. 1 a were prepared using the method shown in Fig. 7b above. BR 2000 elastic laminate was used as the web 200 of an extensible material 22. The web of BR 2000 elastic laminate exhibited a direction of extensibility in CD (i. e. normal to the direction of the moving web). The sealing unit 350 was a mechanical clamping device applying two clamps to the reinforced region 15 of each side panel 10 in order to secure the second (upper) layer 31 to the first (lower) layer 13. Each clamp had a length of about 8 mm and a width of about 1 mm and was applied to the reinforced region 15 with its length being arranged in MD, i. e. in the direction of the running web 200 and normal to said direction of extensibility of the web. The clamps were applied essentially symmetrical in a distance of each about 1 - 1.5 cm with respect to the center of the side panel 10.

A 25 mm x 65 mm sample was cut from the reinforced region 15 as was described above whereby such sample included the two clamps. The sample was then inserted into the Zwick tensile tester.

The stress-strain (or force-elongation) curve obtained is shown in Fig. 9 (broken lines); the upper curve corresponds to the first upload, the lower curve to the second upload. The strain (retraction force) measured at an elongation of 100 % was about 8.3 N for the first upload and 7.8 N for the second upload.

### Comparative Example 1

A 25 mm x 65 mm sample was cut from the first (lower) layer 30 of the side panel 10 outside of the reinforced region 15 by applying a cut having the same geometry and orientation as the cut used to obtain the sample of the reinforced region 15. The sample was mounted into the Zwick tensile tester as was described above.

The stress-strain (or force-elongation) curve obtained is shown in Fig. 9 (solid lines); the upper curve corresponds to the first upload, the lower curve to the second upload. The strain (retraction force) measured at an elongation of 100 % was about 4.25 N for the first upload and 4.0 N for the second upload.

### Example 2

Extensible side panels 10 of the type of Fig. 1 a were prepared using the method shown in Fig. 9 above. COBAN™ elastic laminate was used as the web 200 of an extensible material. The web of COBAN elastic laminate exhibited a direction of extensibility in MD (i. e. in the direction of the moving web). The sealing unit 350 was a mechanical clamping device applying two clamps to the reinforced region 15 of each side panel 10 in order to secure the second (upper) layer 31 to the first (lower) layer 13. Each clamp had a length of about 8 mm and a width of about 1 mm and was applied to the reinforced region 15 with its length being arranged in MD of the side panel, i. e. normal to said primary direction of extensibility of the side panel. The clamps were applied essentially symmetrical in a distance of each about 1 - 1.5 cm with respect to the center of the side panel 10.

A 25 mm x 65 mm sample (MD x CD) was cut from the reinforced region 15 as was described above so whereby such sample included the two clamps. The sample was then inserted into the Zwick tensile tester.

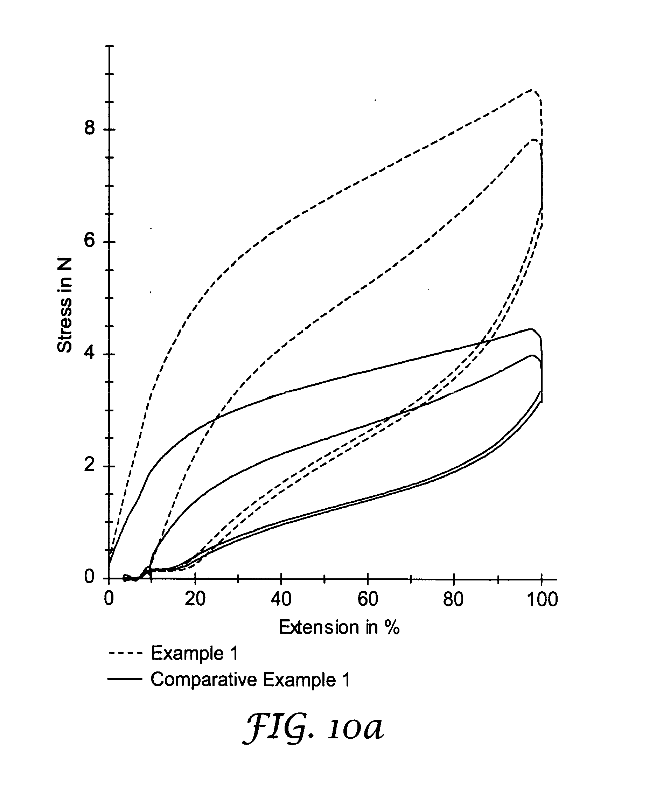

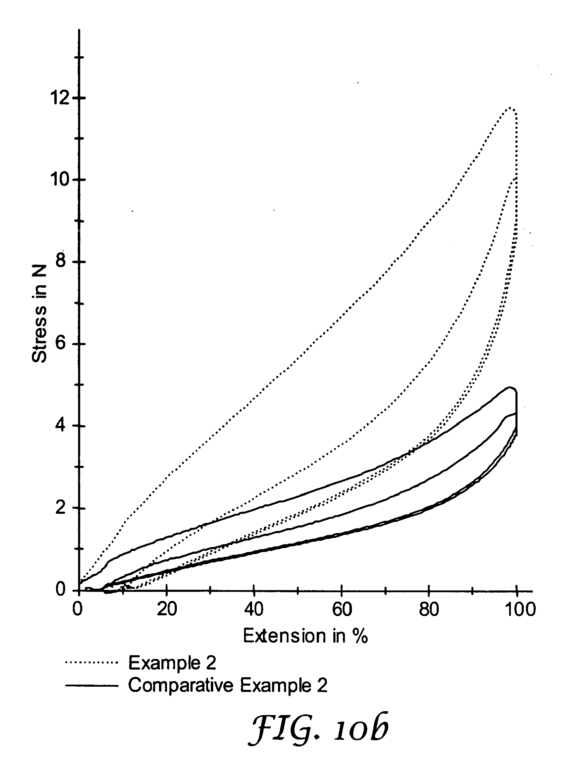

The stress-strain (or force-elongation) curve obtained is shown in Fig. 10 (broken lines); the upper curve corresponds to the first upload, the lower curve to the second upload. The strain (retraction force) measured at an elongation of 100 % was about 11,75 N for the first upload and 10,0 N for the second upload.

### Comparative Example 2

A 25 mm x 65 mm sample was cut from the first (lower) layer 30 of the side panel 10 outside of the reinforced region by applying a cut having the same geometry and orientation as the cut used to obtain the sample of the reinforced region 15. The sample was then mounted into the Zwick tester as was described above.

The stress-strain (or force-eiongation) curve obtained is shown in Fig. 10 (solid lines); the upper curve corresponds to the first upload, the lower curve to the second upload. The strain (retraction force) measured at an elongation of 100 % was about 5.0 N for the first upload and 4.25 N for the second upload.

### List of reference numbers

- 10: extensible side panel
- 11: first (left) longitudinal side edge of the extensible side panel
- 12: second (right) longitudinal side edge of the extensible side panel
- 13: first top lateral edge
- 14: second bottom lateral edge
- 15: extensibly reinforced region
- 16: fastening member
- 17: support layer
- 18: hypothetical cut line
- 19: hypothetical region
- 21: fingerlift
- 22, 22': extensible material of first and second layer 30, 31, respectively
- 23: adhesive layer
- 24: welded attachment lines
- 25: fastening tabs
- 30: first layer comprising an extensible material 22
- 30a: top lateral edge of second layer 30
- 31: second layer comprising an extensible material 22
- 31 a: top lateral edge of second layer 31
- 32: top lateral edge of extensibly reinforced region
- 100: disposable absorbent article
- 101: top sheet
- 102: back sheet
- 103: absorbent core
- 104: first longitudinal edge of disposable absorbent article
- 105: second longitudinal edge of disposable absorbent article
- 106: back waistband region
- 107: front waistband region
- 108: landing zone
- 109: attachment region
- 110: chassis of diaper

- 200: web comprising a web of an extensible material 22
- 200', 200", 200^{(x')},...200^{(n')}: sub-web comprising a web of an extensible material 22
- 201: first (left) longitudinal side edge of web 200
- 202: second (right) longitudinal side edge of web 200
- 210: designated fold line
- 211: intermediate cut line
- 212: foldable flap
- 213: designated cut lines
- 216: continuous web or discontinuous sequence of fastening members 16
- 217: web of support layer 17
- 218: centre line of web 200
- 219: cut line through continuous web or discontinuous sequence 216 of fastening member 16
- 230: web of an extensible material 22
- 250: supply roll of web 200
- 251, 251': roller
- 300, 300': rotary die cut unit
- 301, 301': cut roller
- 302, 302': pressure roller
- 305: sinusoidally shaped cutting knives
- 310: folding device
- 311: folding bracket
- 312: nozzle supplying pressurized air
- 313: sprocket wheel
- 350: sealing unit
- 400, 400': vacuum belt
- 500: vacuum wheel
- 501: turning device
- 502: folding bracket
- 600: storage container

- 700: separation unit

## Claims

1. An extensible side panel (10) for use in disposable articles such as a diaper (1), said panel comprising a first longitudinal side edge (11), a second longitudinal side edge (12), a first top lateral edge (13) and a second bottom lateral edge (14), the side panel comprising a first layer (30) of an extensible material (22) having a top lateral edge (30a) and a second layer (31) of an extensible material (22') having a top lateral edge (31 a), said second layer (31) being superimposed onto said first layer (30) to provide at least one extensibly reinforced region (15), the top lateral edge or edges (31 a) of the second layer (31) being arranged essentially parallel to the top lateral edge (30a) of the first (lower) layer of the side panel (10).

2. An extensible side panel (10) according to claim 1 wherein the top lateral edge or edges (31 a) of the second layer (31) are arranged essentially adjacent to the top lateral edge or edges (30a) of the first layer (30) of the side panel (10).

3. An extensible side panel (10) according to claim 2 wherein the top lateral edge or edges 30a of the first layer 30, the top lateral edge or edges 31 a of the second layer 31 and the first top lateral edge of the side panel 13 essentially coincide.

4. An extensible side panel (10) according to any of the preceding claims wherein the reinforced region (15) has a primary direction of extensibility essentially in the direction of the first top lateral edge (13), in the direction of the top lateral edge (30a) of the first layer (30) and/or the in cross-direction.

5. An extensible side panel (10) according to any of the preceding claims wherein the first layer (30) and the second layer (31) comprise the same extensible material (22).

6. An extensible side-panel (10) according to any of the preceding claims wherein the first layer (30) of an extensible material (22) has a direction of extensibility in the cross-direction and/or in the direction of the top lateral edge (30a).

7. An extensible side panel (10) according to any of the preceding claims wherein the sum of the extensions of the top lateral edge or edges 31 a of the second layer or layers (31) is at least about 30 % of the extension of the first top lateral edge (13) and/or of the extension of the top lateral edge(s) 30a.

8. An extensible side panel (10) according to any of the preceding claims wherein the extensibly reinforced region (15) provides a retraction force upon extending such area by an extension of about 50 % essentially in the direction of the first top lateral edge (13), in the direction of the top lateral edge (30a) of the first layer (30) and/or in the cross-direction which is at least 25 % higher in comparison to the retraction force of a hypothetical side panel having no second layer (31) of an extensible material (22).

9. An extensible side panel (10) according to any of the preceding claims wherein the elastically reinforced region (15) is equal to or less than the hypothetical region (19) formed by the second bottom lateral edge (14), a hypothetical cut line (18) which is parallel to the first longitudinal edge (11) and extends through the lowest point of the second bottom lateral edge (14), and optionally a hypothetical extension of the first and/or second longitudinal edge (11) and (12).

10. An extensible side panel according to claim 9 wherein the area of the extensibly reinforced region (15) is at least about 50 % of the area of the hypothetical region (19).

11. An extensible side panel (10) according to any of the preceding claims having at least one fastening member (16) attached along at least one of the longitudinal edges (12) and/or (13).

12. An extensible side panel (10) according to claim 11 wherein the fastening member (16) comprises a first mechanical closure element engagable with a complimentary second closure element.

13. An extensible side panel (10) according to any of the preceding claims wherein the extensible material (22) is selected from a group of materials comprising film based elastics, elastic base films with single sided or double-sided non-woven skin laminates in complete or intermitted bonding pattern, elastic film based elastics in any breathable form with non-woven skin laminates, elastic filament or elastic net based constructions with non-woven surface laminates.

14. A disposable absorbent article (100) such as a diaper, a sanitary napkin or a training pant comprising a liquid permeable top sheet (101), a liquid-impermeable back sheet (102) opposite to said top sheet (101), a liquid-absorbent core (103) between said top sheet (101) and said back sheet (102), longitudinal edges (104) and (105) and at least one extensible side panel (10) according to any of claims 1 - 13 which is attached along at least one of the longitudinal edges (104) and/or (105).

15. A method for preparing an extensible side panel (10) according to any of claims 1 - 13 comprising
(a) providing a web (200) in a machine direction comprising a layer (30) of an extensible material (22) and a plurality of designated fold lines (210), said web having longitudinal side edges (201) and (202) and said layer (30) having a direction of extensibility essentially in the cross-direction,
(b) making a series of intermediate cut lines (211) in said web (200) along a portion of the designated fold lines (210) while maintaining its integrity to provide foldable flaps (212) adjacent to the designated fold lines (210),
(c) folding over said flaps (212) around said designated fold lines (210) and attaching the flap (212) to the underlying web (200) to provide extensibly reinforced regions (15), and
(d) cutting said web (200) along the uncut portion of said designated fold lines (210) to provide individual extensible side panels (10).

16. A method according to claim 15 comprising providing at least one continuous or discontinuous sequence (216) of fastening members (16) in the machine direction of said web (200), said method comprising the step of cutting said web (200) through said sequence (216) of fastening members (16) to provide two separate webs (200', 200") each bearing a sequence of fastening members (16) along one of its longitudinal edges (201', 202').

17. Method according to claim 16 wherein said sequence (216) of fastening members (16) extends essentially along the centre line (217) of said web (200).

18. A method for preparing an extensible side panel (10) according to any of claims 1 - 13 comprising
(a) providing a web (200) in a machine direction comprising a layer (30) of an extensible material (22), a plurality of longitudinal designated fold lines (210), longitudinal side edges (201) and (202) and a plurality of designated cut lines (213), said layer (30) having a direction of extensibility essentially in the machine direction,
(b) making a series of intermediate cut lines (211) in said web (200) along portions of the designated fold lines (210) while maintaining the integrity of the web (200) to provide foldable flaps (212) adjacent to the designated fold lines (210),
(c) folding over said flaps (212) around said designated fold lines (210) and attaching the flap (212) to the underlying web (200) to provide the extensibly reinforced regions (15),
(d) cutting said web (200) along the remaining portions of said designated fold lines (210) to provide a plurality of individual webs (200', 200" ... 200 ^{(n')}), and
(e) cutting said individual webs (200') along said designated cut lines (213) in the cross direction to provide individual extensible side panels (10).

19. A method according to claim 18 comprising providing a continuous or discontinuous sequence (216) of fastening members (16) distributed essentially along the designated cut lines (213) in the cross-direction of said web (200) so that said closure components (216) are cut in cross-direction when cutting said individual webs (200', 200" ... 200 ^{(n')}) along said designated cut lines (213).

20. A method for preparing an extensible side panel (10) according to any of claims 1 -13 comprising
(a) providing a web (200) in a machine direction comprising a layer (30) of an extensible material (22) and a plurality of designated first top lateral edges (13), said web (200) having longitudinal side edges (201) and (202) and said layer (30) having a direction of extensibility in the cross-direction,
(b) making a series of intermediate cut lines (211) in said web (200) along said designated first top lateral edges (13) to provide a series of individual precursors of the extensible side panel comprising foldable flaps (212) extending between the intermediate cut lines (211) and the designated first top lateral edges (13) and
(c) folding over said flaps around the designated first top lateral edge (13) and attaching the flap (212) to the first underlying layer (30).

21. A method according to claim 20 wherein the precursors are rotated subsequent to step (b) around an axis normal to said layer (30) so that the foldable flap can be folded over in the cross-direction.
